# EUROPEAN PATENT SPECIFICATION

(11) **EP 3 181 682 B1**
(45) Date of publication and mention of the grant of the patent: **21.04.2021**
(21) Application number: 17154984.3
(22) Date of filing: 29.09.2009
(51) Int. Cl.: C12N 1/00

(54) **REDUCED COLONIZATION OF MICROBES AT THE MUCOSA**
REDUZIERTE MIKROBENKOLONISIERUNG AN DER SCHLEIMHAUT
COLONISATION RÉDUITE DE MICROBES AU NIVEAU DE LA MUQUEUSE

(30) Priority: 29.09.2008 EP 08105458
(43) Date of publication of application: 21.06.2017
(62) Divisional of application: 09736880.7
(73) Proprietor: Intrexon Actobiotics NV, 9052 Zwijnaarde (BE)
(72) Inventor: STEIDLER, Lothar, 9160 Lokeren (BE)
(74) Representative: Pohlman, Sandra M.

(56) References cited:
- WO-A-02/090551
- WO-A-2005/111194
- STEIDLER LOTHAR ET AL: "Therapeutic drug delivery by genetically modified Lactococcus lactis", ANNALS OF THE NEW YORK ACADEMY OF SCIENCES, NEW YORK ACADEMY OF SCIENCES, NEW YORK, NY, US, 3 September 2005 (2005-09-03), pages 176-186, XP009101905, ISSN: 0077-8923
- BIGAS ANNA ET AL: "Colonization capacity and serum bactericidal activity of Haemophilus parasuis thy mutants", INTERNATIONAL MICROBIOLOGY, SPRINGER VERLAG IBERICA, BARCELONA, ES, vol. 9, no. 4, 1 December 2006 (2006-12-01), pages 297-301, XP009101779, ISSN: 1139-6709
- YU FENGGANG ET AL: "Construction and characterization of a thyA mutant derived from cholera vaccine candidate IEM101.", MOLECULAR BIOTECHNOLOGY MAR 2005, vol. 29, no. 3, March 2005 (2005-03), pages 191-196, XP002558411, ISSN: 1073-6085

## Description

The present invention relates to recombinant non-pathogenic and non-invasive Gram-postive bacteria that, in comparison to the wild type ancestor, show reduced capacity of colonization to the mucosa, in particular when residing in the alimentary tract of a human or animal. In particular, the invention relates to the engineering and use of colonizing non-pathogenic and non-invasive Gram-postive bacteria with reduced colonizing capacity, e.g. as host organisms for expression and delivery of therapeutic proteins and peptides.

### Field of the Invention

The invention is in the field of use of colonizing non-pathogenic and non-invasive Gram-postive bacteria (such as, for example, *Lactobacillus* sp, *Streptococcus* sp., *Bacteroides* sp., *Bifidobacterium* sp., ...) in human and veterinary medicine. Medical applications can be a consequence of inherent features of such bacteria, or such bacteria can be genetically modified (GM) for the administration of prophylactic or therapeutic peptides or proteins to humans or animals suffering from or at a risk of developing a disease, or such GM bacteria may be used for vaccination purposes.

More in particular, the invention relates to GM bacteria that show reduced capacity of colonization, in particular when residing in the human or animal mucosa (such as, without limitation, alimentary tract, oral cavity, nasal cavity, urogenital tract, etc.) as part of a treatment or vaccination of a human or animal.

In a preferred embodiment, the GM bacterium contains an inactive thymidylate synthase gene that causes the reduced capability for said bacterium to colonize in human or animal mucosa. In a more preferred embodiment, the GM bacterium contains an inactive thymidylate synthase gene that completely prevents the GM bacterium to colonize in human or animal mucosa .

### Background of the Invention

Both wild type as well as GM microbes are gaining importance in medical applications in humans and animals where applications using prophylactic as well as therapeutic microbes are described in pre-clinical and clinical settings.

Selected microbes (also called functional microflora, probiotics) can be used for the prevention or alleviation of pathologies.

Lactic acid bacteria but also other species are increasingly becoming important as hosts for recombinant expression of heterologous polypeptides *in vitro* (e.g., US 5,559,007) as well as for *in vivo* or *in situ* expression and delivery of antigens and/or therapeutically relevant polypeptides (e.g., WO 97/14806).

Lactic acid bacteria, and particularly *Lactobacillus* and *Lactococcus,* are considered as GRAS-microorganisms (i.e., generally regarded as safe) and may thus be relatively readily administered to humans and animals.

Because *Lactococcus lactis* was the first lactic acid bacteria species characterised into great detail at a molecular and genetic level, it enjoyed an increasing interest as production host for heterologous proteins and eventually as *in situ* delivery system for biologically active molecules. *L. lactis* is non-pathogenic, non-invasive and non-colonizing.

Compared to expression systems in non-colonizing microorganisms however, the use of colonizing microorganisms may facilitate protein expression and delivery *in situ,* via prolonged retention and closer contact to the host tissues. The potency of any therapeutic application may therefore benefit substantially from using a colonizing microorganism.

As an example, the more robust character of the *Lactobacillus* bacteria may prefer its use as a medicament over the *Lactococcus* bacteria.

However, the disadvantage of using colonizing instead of non-colonizing microbes (such as using *Lactobacillus* sp instead of *Lactococcus*) as a host strain for delivering therapeutic proteins or peptides to human or animal is the unwanted colonization of the mucosa, in particular of the alimentary tract, by the prophylactic or therapeutic strain, which makes it rather difficult to completely stop the treatment after cure (or in case of side effects) or to establish a dose response relationship. Indeed, timing and dosage will intrinsically be more difficult to perform when using a colonizing microorganism as when compared to using a non-colonizing microorganism. Sometimes, the only possibility to stop the treatment may be the use of selective antibiotics, which should however be avoided due to development of antibiotic resistance, impact on other microflora and other reported side effects. Moreover, it may be more difficult to environmentally contain a colonizing microorganism as it will be unclear for how long the microorganisms will remain attached to the mucosal surface, which complicates the regulatory and biosafety considerations with such organisms.

Said disadvantages clearly complicate the use of non-recombinant selected microbes and has prevented until now that recombinant colonizing strains have been used and approved for clinical trials. For these reasons, colonizing microorganisms with reduced colonization capacity could hold even more advantages, including more precise control on timing and dosage of protein expression, as well as a clear view on residence time and environmental containment, which are considered critical factors for deliberate release of any genetically modified organisms (GMO).

A few authors have investigated the effect of a thymidylate synthase gene (ThyA) mutation on the colonization capacity of bacteria (Bigas et al., 2006 (Int Microbiol 9(4): 297-301) (Besier et al., 2007 (Int J Med Microbiol 297(4): 217-25). The results of these studies are contradictive and only relate to pathogenic bacteria and the relation of ThyA to the remaining pathogenicity. In clinical settings one has observed even that thymidine-auxotrophic small colony variants of *Staphylococcus aureus* are able to persist in patients suffering from chronic airway infections and have been implicated in persistent, relapsing and treatment-resistant infections (Proctor et al., 1995 (Clin Infect Dis 20(1): 95-102) (Spanu et al., 2005 (Clin Infect Dis 41(5): e48-52). Besier et al (2007) has even shown that defects in the ThyA function are causative for the formation of the thymidine-auxotrophic small colony variants of *Staphylococcus aureus.* Another author, Hasselbring et al. (Hasselbring et al., 2006 (J Bacteriol 188(17): 6335-45), has screened *Mycoplasma pneumonia* mutants to identify genes associated with gliding but dispensable for cytadherence, which resulted in a number of genes (including ThyA) suggesting to be involved in the gliding motility of *Mycoplasma pneumonia.*

Other authors have used ThyA gene mutations for biological containment of genetically modified *Lactococcus sp.* and *Lactobacillus sp.,* in order to avoid the surviving and spreading of the genetically modified organisms in the environment (Steidler et al., 2003 (Nat. Biotechnol. 21(785-9), WO2004/046346, WO2005/111194). The experiments in these disclosures are mainly related to growth and survival of said recombinant bacteria and did not yield any information on the colonization capacity of the *Lactobacillus* ThyA mutants nor did they give any direction to a possible use of ThyA gene impairment in *Lactobacillus* to reduce or avoid colonization. Instead, given that some of these publications indicated the ability of *Lactobacillus* ThyA mutants to produce and deliver therapeutic proteins *in situ,* one would have no reason to expect said bacteria to display reduced colonization.

### Summary of the Invention

It is the objective of the present invention to obtain a suitable system to reduce or avoid the colonization capacity of prophylactic and therapeutic, non-pathogenic and non-invasive Gram-postive bacteria at the mucosa, in particular in the alimentary tract.

We have surprisingly found that a defect in the function of ThyA in otherwise colonizing bacteria (e.g. *Lactobacillus sp., Streptococcus* sp. or *Bacteroides* sp.) causes a reduced colonization capacity and in a preferred setting a complete prevention of colonization of these strains in the mucosa (in particular in the alimentary tract). Hence, rendering defective a thyA gene of a microorganism, such as an endogenous thyA gene and more particularly an endogenous chromosomally-located thyA gene of such a bacterium, allows to reduce or abolish the latter's colonizing capacity.

The invention provides a non-pathogenic, non-invasive Gram-positive bacterium comprising:
(a) a defective thymidylate synthase (*thyA*) gene;
(b) a recombinant nucleic acid comprising a promoter operably linked to one or more open reading frames (ORFs) encoding one or more heterologous expression products which are capable of eliciting a prophylactic and/or therapeutic response in a human or animal,
wherein said bacterium has a reduced capacity of colonizing a mucosa of said human or animal, in comparison to its wild type ancestor, and
wherein said promoter in the recombinant nucleic acid is the native promoter of a *Lactococcus* gene selected from the group consisting of: glyceraldehyde-3-phosphate dehydrogenase (*gapB*), non-heme iron-binding ferritin (*dpsA*), pyruvate kinase (*pyk*), phosphopyruvate hydratase (*eno*), dipeptidase PepV (*pepV*), superoxide dismutase (*sodA*), ribosomal protein L19 (*rplS*), HU-like DNA binding protein (*hllA*), and 50S ribosomal protein L28 (*rpmB*).

The invention also provides a method for reducing or abolishing the colonization capacity of a bacterium, wherein said bacterium is capable of residing in the mucosa of a human or animal and wherein said bacterium comprises a recombinant nucleic acid which encodes one or more heterologous expression products capable of eliciting a prophylactic and/or therapeutic response in said human or animal, wherein said recombinant nucleic acid comprises a promoter which is a native promoter of a *Lactococcus* gene selected from the group consisting of: glyceraldehyde-3-phosphate dehydrogenase (*gapB*), non-heme iron-binding ferritin *(dpsA),* pyruvate kinase (*pyk*), phosphopyruvate hydratase (*eno*), dipeptidase PepV (*pepV*), superoxide dismutase (*sodA*), ribosomal protein L19 (*rpIS*), HU-like DNA binding protein (*hllA*), and 50S ribosomal protein L28 (*rpmB*), wherein said method comprises rendering defective a thymidylate synthase (*thyA*) gene in said bacterium, and wherein said one or more heterologous expression products is selected from: insulin, a growth hormone, prolactin, calcitonin, luteinising hormone, parathyroid hormone, somatostatin, thyroid stimulating hormone, vasoactive intestinal polypeptide, a cytokine, a receptor or antagonist thereof, hIL-10, GLP-2, GLP-1, TFF or hPYY.

Described herein is an isolated strain of a microbe comprising a defective thymidylate synthase (thyA) gene. Preferably, the thyA gene may be rendered defective by means of recombinant DNA technology, such that the defective thyA gene may be denoted as recombinant defective thyA gene, and by extension a recombinant microbe. Preferably disclosed is the isolated strain of the microbe comprising the defective thyA gene, and in particular the defective recombinant thyA gene, wherein said isolated strain of the microbe has a reduced capacity of colonizing a mucosa (e.g., one or more mucosa loci) of a human or animal in comparison to the wild-type microbe, such as a wild-type ancestor of the isolated strain.

Preferably, said defective thyA gene, and particularly said defective recombinant thyA gene, is situated in the chromosome and is inactivated by gene disruption. Preferably, said defective thyA gene, and particularly said defective recombinant thymidylate synthase gene, is a non-reverting mutant gene.

Further preferably, the isolated strain of the microbe comprising the defective thyA gene as taught herein may be a thymidylate synthase deficient strain, i.e., the strain may be deficient in thymidylate synthase activity, such as for example may display a substantial reduction in or more preferably absence of thymidylate synthase activity. Hence, phenotypically said isolated strain may preferably manifest as a thymine and/or thymidine auxotrophic strain, i.e., requiring exogenously provided thymine and/or thymidine for survival, growth and/or propagation. Hence, preferably in the isolated strain of the microbe comprising the defective thyA gene as taught herein, said defect in the thyA gene and consequently in thymidylate synthase activity is not complemented by another, active (non-defective) thyA gene.

Further preferably, the isolated strain may be derived from a microbe which is normally (i.e., when thymidylate synthase proficient, in other words when comprising an active thyA gene) colonizing, i.e., capable of colonizing a mucosa, such as human or animal mucosa, preferably mucosa or the alimentary tract.

As described herein, the microbe may be a yeast or fungi, particularly a recombinant yeast or fungi, in particular any yeast or fungi capable of surviving in the mammalian intestine. Alternatively, said yeast or fungi may have a known probiotic capacity, such as without limitation yeast or fungi strains selected from kefir, kombucha or dairy products.

Said yeast or fungi, particularly said recombinant yeast or fungi, may be selected from the group consisting of *Candida sp., Aspergillus sp., Penicillium sp., Saccharomyces sp., Hansenula sp., Kluyveromyces sp. Schizzosaccharomyces sp. Zygosaccharomyces sp., Pichia sp., Monascus sp., Geotrichum sp* and *Yarrowia sp.* More in particular, said yeast is *Saccharomyces cerevisiae,* even more in particular said yeast is *Saccharomyces cerevisiae* subspecies *boulardii.*

The isolated strain comprising defective thyA gene as taught herein may be of a microbe which is a bacterium, more preferably a non-pathogenic and/or non-invasive bacterium, yet more preferably a Gram-positive bacterium, in particular such bacterium capable of residing in mucosa of human or animal, in particular in the mucosa of the alimentary tract, such as a normally (i.e., when thymidylate synthase proficient) colonizing bacterium. Exemplary bacterial species include without limitation, *Bacteroides sp, Clostridium sp., Fusobacterium sp., Eubacterium sp., Ruminococcus sp., Peptococcus sp., Peptostreptococcus sp., Streptococcus* sp., *Bifidobacterium sp., Escherichia sp.,* and *Lactobacillus sp.* Another aspect of the invention is an isolated strain of a gram-positive bacterium that is capable of residing in the mucosa, in particular in the mucosa of the alimentary tract, such as *Bacteroides sp, Clostridium sp., Eubacterium sp., Ruminococcus sp., Peptococcus sp., Peptostreptococcus sp., Streptococcus sp., Bifidobacterium sp.* and *Lactobacillus sp.,* wherein such gram-positive bacterium comprises a defective thyA gene, in particular a defective recombinant thymidylate synthase gene (thyA). Preferably, said defective thyA gene, and particularly said defective recombinant thyA gene, is situated in the chromosome and inactivated by gene disruption. Preferably, said defective thyA gene, and particularly said defective recombinant thymidylate synthase gene, is a non-reverting mutant gene.

In a preferred embodiment the bacterium is an isolated strain of *Lactobacillus sp.* The defective thyA gene may be, in particular a defective recombinant thymidylate synthase gene (thyA). Preferably, said defective thyA gene, and particularly said defective recombinant thyA gene, is situated in the chromosome and is inactivated by gene disruption. Preferably, said defective thyA gene, and particularly said defective recombinant thymidylate synthase gene, is a non-reverting mutant gene. Preferably, said *Lactobacillus sp.* is a *Lactobacillus plantarum* strain, *Lactobacillus acidophilus* or *Lactobacillus rhamnosus* strain; even more preferably, said *Lactobacillus* is a *Lactobacillus salivarius* strain or a *Lactobacillus casei* strain; and any subspecies and strains thereof.

In a preferred embodiment the bacterium is an isolated strain of *Bacteroides sp.* The defective thyA gene may be, in particular a defective recombinant thymidylate synthase gene (thyA). Preferably, said defective thyA gene, and particularly said defective recombinant thyA gene, is situated in the chromosome and is inactivated by gene disruption. Preferably, said defective thyA gene, and particularly said defective recombinant thymidylate synthase gene, is a non-reverting mutant gene. *Bacteroides sp.* are highly suitable for delivery of prophylactic and/or therapeutic molecules such as proteins to subjects (see, e.g., (Hamady et al., 2009 (Gut) Epub ahead of print). Preferably, said *Bacteroides sp.* is a *Bacteroides ovatus* strain, and any subspecies and strains thereof.

In a further preferred embodiment the bacterium is an isolated strain of *Streptococcus sp.* The defective thyA gene may be, in particular a defective recombinant thymidylate synthase gene (thyA). Preferably, said defective thyA gene, and particularly said defective recombinant thyA gene, is situated in the chromosome and is inactivated by gene disruption. Preferably, said defective thyA gene, and particularly said defective recombinant thymidylate synthase gene, is a non-reverting mutant gene. Preferably, said *Streptococcus sp.* is *Streptococcus mutans.* The bacterium, strain or host cell as intended herein may preferably be a *Streptococcus sp* more preferably *Streptococcus mutans* and any subspecies and strains thereof, comprising a defective thyA gene.
*Streptococcus mutans* is a lactic acid bacterium that normally colonizes dental surfaces, and as such an may be particularly suitable to serve as a host organism for delivery of molecules as taught herein to the oral mucosa.
*S*. *mutans* constitutes a phenotypically homogeneous group of colonizing, Gram-positive *Streptococci* (Hamada et al., 1980 (Microb rev 44(2): 331-84). *S. mutans* is acidogenic and acidoduric, non-motile and facultative anaerobic. At present, *S. mutans* is commonly divided into four serotypes, based on the chemical composition of its cell surface rhamnose-glucose polymers (Hamada et al., 1980 (Microb rev 44(2): 331-84; Maruyama et al., 2009 (BMC genomics 10(358). In this view, serotype c is dominant among *S. mutans* clinical isolates (almost 80%) and is now considered the ancestral phenotype, with other serotypes having evolved strain-specific genes (Maruyama et al., 2009 (BMC genomics 10(358).The natural habitat of *S. mutans* is the human mouth, with a clear preference for tooth surfaces as well as prosthetic devices (Hamada et al., 1980 (Microb rev 44(2): 331-84). The organism can also be isolated from feces, in humans (Kilian et al., 1971 (Archives of oral biology 16(5): 553-4; Finegold et al., 1975 (Can res 35(11 Pt. 2): 3407-17; Liljemark et al., 1978 (J dent resh 57(2): 373-9; Hamada et al., 1980 (J clin microbiol 11(4): 314-8; Unsworth, 1980 (J hyg 85(1): 153-64) as well as rats (Huber et al., 1977 (J of dent res 56(12): 1614-9; Thomson et al., 1979 (Caries res 13(1): 9-17). Although the bacterium appears not to be widely distributed in wild animals, *S. mutans* has among others been isolated from oral surfaces of several monkey and bat species (Lehner et al., 1975 (Nature 254(5500): 517-20; Coykendall et al., 1976 (Infection and immunity 14(3): 667-70; Dent et al., 1978 (Journal appl bact 44(2): 249-58; Beighton et al., 1982 (Archives of oral biology 27(4): 331-5), rats (Lehner et al., 1975 (Nature 254(5500): 517-20; Coykendall et al., 1976 (Infection and immunity 14(3): 667-70; Hamada et al., 1980 (Microbio rev 44(2): 331-84), hamsters (Gehring et al., 1976 (Deutsche zahnarztliche Zeitschrift 31(1): 18-21; Hamada et al., 1980 (Microb rev 44(2): 331-84), macropods (Samuel, 1982 (Archives of oral biology 27(2): 141-6) and *Beagle* dogs (Wunder et al., 1976 (J dent res 55(6): 1097-102).
The oral microflora is a complex ecosystem of microbial species, and without proper oral hygiene, large microbial masses and biofilms (plaques) may develop on dental surfaces. Although the causative relationship between specific oral bacterial species and dental caries has been the subject of many studies, *Streptococci* normally comprise the majority of the total viable cell count retrieved from human carious lesions (Hamada et al., 1980 (Microb rev 44(2): 331-84).
The adherence of *S. mutans* and other oral bacteria to tooth surfaces and the formation of dental plaque are of major significance in the development of dental caries. These processes are complex and involve a variety of bacterial and host components. Bacterial attachment to the tooth surface is usually preceded by the formation of a thin layer of heterogeneous salivary glycoproteins (pellicle), which facilitates the adhesion of *S. mutans.*
*S. mutans* is capable of metabolizing (fermenting) a wide variety of carbohydrates. The acidification of the local environment by the end product of metabolism (i.e., lactic acid) inhibits many competing bacterial species, thus enabling *S. mutans* to maintain its niche, while at the same time causing dental demineralization in the host. Importantly, while the fermentation of any available carbohydrate could lead to lactic acid and damage to the dental enamel, sucrose is particularly important in this process because it also serves as substrate for extracellular enzymes which synthesize sucrose-derived polymers. These extracellular polymers (glucans) consist solely of glucose units and possess a marked ability to promote adherence when synthesized *de novo* on various solid surfaces (Hamada et al., 1980 (Microb rev 44(2): 331-84).
Several approaches to reduce *S. mutans* colonization have been described, focusing mainly on dental plaque-related diseases. These interventions range from the obvious mechanical cleansing, to compounds targeting bacterial interactions, the salivary pellicle or bacterial polymers adsorbed to the tooth (i.e. glucans), as well as strategies aimed at therapeutic manipulation of the oral microflora (Liljemark et al., 1978 (J dent res 57(2): 373-9; Allaker et al., 2009 (Int J Antimicrob Agents 33(1): 8-13), such as for example using probiotics such as specific *Lactobacillus* strains, or using replacement therapy with less cariogenic or even impaired *S. mutans* strains, such as for example via deletion (and if necessary, compensation) of the genetic sequence encoding lactate dehydrogenase (e.g., WO/1996/040865) (Allaker et al., 2009 (Int J Antimicrob Agents 33(1): 8-13). Such clones produce no detectable lactic acid and are significantly less cariogenic. In this view however, the genetic modification is to maintain or even improve upon the colonizing potential of the engineered *S. mutans* strains, to replace the wild type *S. mutants* bacteria, which is distinct from the goal of the present invention, i.e., to obtain strains such as *S. mutans* strains having reduced or abolished colonization capacity.

A "non-reverting mutant" as used throughout this specification means that the reversion frequency is lower than 10⁻⁸, preferably the reversion frequency is lower than 10⁻¹⁰, even more preferably, said reversion frequency is lower than 10⁻¹², even more preferably, said reversion frequency is lower than 10⁻¹⁴, most preferably, said reversion frequency is not detectable using the routine methods known to the person skilled in the art.

The main advantage of the invention is the improved use of the selected or recombinant microbes (e.g. *Lactobacillus sp., Bacteroides sp.,* or *Streptococcus* sp.) as a medicament to treat animals or humans suffering from a disease, or its use as a vaccine, to the extent that it is now possible to have a controlled dosing of the prophylactic and/or therapeutic microbes. This can avoid the use of antibiotics in case of terminating the treatment.
Prophylactic and/or therapeutic traits may be inherent to the strain comprising the defective ThyA (e.g., probiotic microbes) and/or may be expressed as a consequence of genetic engineering of the strain comprising a defective ThyA .
Accordingly, an isolated strain of a microbe comprising the defective thyA gene as taught herein, may elicit a prophylactic and/or therapeutic effect in a subject, preferably in a human or animal. Also provided is an isolated strain of a microbe comprising the defective thyA gene as taught herein, wherein the strain further expresses an expression product, preferably a heterologous expression product, particularly a prophylactically and/or therapeutically relevant expression product (such as an expression product capable of eliciting a prophylactic and/or therapeutic response in a subject, preferably in a human or animal subject), such as for example a (preferably, heterologous) peptide, polypeptide or protein, and more particularly antigens and/or non-vaccinogenic prophylactically and/or therapeutically active peptides, polypeptides or proteins. To achieve the expression of said heterologous expression product(s), the strain may commonly comprise a recombinant nucleic acid encoding the heterologous expression product(s). Advantageously, the recombinant nucleic acid may comprise suitable regulatory sequence(s) (e.g., a promoter) operably linked to one or more open reading frames encoding the heterologous expression product(s).

Therefore, another aspect of the invention is the use of an isolated strain of the microbe comprising the defective thyA gene as taught herein, and particularly the use of *Lactobacillus* strain, *Streptococcus* strain (e.g., *Streptococcus mutans* strain) or *Bacteroides* strain (e.g., *Bacteroides ovatus* strain), comprising the defective thyA gene according to the invention, as a reduced colonizing, or even preferably non-colonizing, strain for the delivery of prophylactic and/or therapeutic molecules, such as for the delivery of one or more (preferably heterologous) expression products as taught herein. Preferably, delivery using a *Streptococcus* strain (e.g., *Streptococcus mutans*) may be to oral mucosa. The delivery of prophylactic and/or therapeutic molecules has been disclosed, as a non-limiting example, in WO 97/14806 and in WO 98/31786. Prophylactic and/or therapeutic molecules include, but are not reduced to polypeptides such as insulin, growth hormone, prolactine, calcitonin, group 1 cytokines, group 2 cytokines, group 3 cytokines, neuropeptides and antibodies (or functional fragments thereof), and polysaccharides such as polysaccharide antigens from pathogenic bacteria. In a preferred embodiment, the thyA gene of an isolated strain of the bacterium, such as particularly of a *Lactobacillus sp.* strain, *Streptococcus* sp. strain or *Bacteroides* sp. strain, preferably *Lactobacillus salivarius* or *Lactobacillus casei,* or *Streptococcus mutans,* or *Bacteroides ovatus,* is disrupted and replaced by a functional human interleukin-10 expression cassette and the strain can be used for delivery of IL-10. Said interleukin-10 expression unit is preferably, but not reduced to, a human interleukin-10 expression unit or gene encoding for human interleukin-10. Therefore, an isolated strain of the microbe comprising the defective thyA gene as taught herein, and particularly the use of a *Lactobacillus sp.* or *Streptococcus* sp. (e.g., *Streptococcus mutans* strain) or *Bacteroides* sp. (e.g., *Bacteroides ovatus* strain),comprising the defective thyA gene strain may be used to deliver human interleukin-10. Methods to deliver said molecules and methods to treat diseases such as inflammatory bowel diseases are explained in detail in WO 97/14806 and WO 00/23471 and Steidler et al. 2000 (Steidler et al., 2000 (Science 289; 1352-5). The present invention demonstrates that the strain according to the invention surprisingly passes the gut at more or less the same speed as the non-colonizing *Lactococcus* strains and show that their loss of colonization capacity results in a much faster clearance of the gut after the last administration than wild type *Lactobacillus sp.*
Another aspect is the use of an isolated strain of the microbe comprising the defective thyA gene as taught herein, and particularly the use of *Lactobacillus* strain or *Streptococcus* strain (e.g., *Streptococcus mutans* strain) or *Bacteroides* strain (e.g., *Bacteroides ovatus* strain), comprising the defective thyA gene according to the invention, as a reduced colonizing probiotic strain, or even preferably non-colonizing probiotic strain.

Another aspect of the invention is a pharmaceutical composition, comprising an isolated strain of the microbe comprising the defective thyA gene as taught herein, and particularly comprising a *Lactobacillus* sp. or *Streptococcus* sp. (e.g., *Streptococcus mutans* strain) or *Bacteroides* sp. (e.g., *Bacteroides ovatus* strain), comprising the defective thyA gene such as a *Lactobacillus* sp. or *Streptococcus* sp. or *Bacteroides* sp. thyA disruption mutant, according to the invention, preferably as a reduced colonizing, or even more preferably non-colonizing strain. The strain may optionally and preferably further express one or more (preferably heterologous) expression products as taught herein. As a non-limiting example, the bacteria may be encapsulated to improve the delivery to the intestine. Methods for encapsulation are known to the person, skilled in the art, and are disclosed, amongst others, in EP0450176. Said pharmaceutical composition may typically comprise one or more suitable excipients, and may further optionally comprise one or more additional active ingredients beneficial for the particular disease or condition to be treated. Further useful compositions comprising the isolated strain of the microbe comprising the defective thyA gene as taught herein may include inter alia starter cultures, innocula, lyophilized compositions, frozen liquid compositions, and food and feed compositions, any of which may comprise additional components common in the art for such compositions.

Still another aspect of the invention is the use of a strain according to the invention, preferably as a reduced colonizing, or even preferably non-colonizing strain, for the preparation of a medicament. Further disclosed is the strain according to the invention, preferably as a reduced colonizing, or even preferably non-colonizing strain, for use as a medicament. Also contemplated is the use of a strain according to the invention, preferably as a reduced colonizing, or even preferably non-colonizing strain, for the preparation of a vaccine. The strain may optionally and preferably further express one or more (preferably heterologous) expression products as taught herein. Hence, also contemplated is the strain as taught herein expressing (and capable of delivering) one or more (preferably heterologous) expression products for use in treating a disease or condition in which the administration of said one or more (preferably heterologous) expression products is capable of eliciting a prophylactic and/or therapeutic effect. Also contemplated is the strain as taught herein expressing (and hence, capable of delivering) one or more (preferably heterologous) expression products for use in delivery of said expression product(s) to a human or animal; and use of said strain for the manufacture of a medicament for delivering said expression product(s) to a human or animal. Preferably, said medicament is used to treat Crohn's disease or inflammatory bowel disease, oral mucositis, lesions of gastro-intestinal tract, autoimmune pathologies, allergy, metabolic disorders such as obesity and diabetes, etc.

Further disclosed is a method for reducing or abolishing the colonization capacity of a microbe, comprising rendering defective a thymidylate synthase (thyA) gene in said microbe. Hereby, the method produces a strain of the microbe having a reduced capacity of colonizing the mucosa of a human or animal in comparison to the wild-type microbe, such as a wild-type ancestor of said strain. Preferred microbe types and species may be as enumerated above. Said rendering defective of the thyA gene may preferably be by means of recombinant DNA technology. Preferably, the thyA gene may be situated in the chromosome and may be rendered defective by gene disruption. Preferably, the thyA gene may be rendered defective non-revertingly.
Preferably, the rendering defective of the thyA gene results in thymidylate synthase deficiency, i.e., a substantial reduction in or more preferably absence of thymidylate synthase activity in the so-modified microbe, such as in particular results in thymine and/or thymidine auxotrophy in said microbe.
Further preferably, the microbe subjected to the method may be normally (i.e., when thymidylate synthase proficient, in other words when comprising an active thyA gene, prior to rendering the same defective) colonizing, i.e., capable of colonizing a mucosa, such as human or animal mucosa, preferably mucosa or the alimentary tract. The microbe may be as taught here above.

Preferably, the microbe may elicit a prophylactic and/or therapeutic effect in a subject, preferably in a human or animal. Preferably, the microbe may express an expression product, preferably a heterologous expression product, particularly a prophylactically and/or therapeutically relevant expression product (such as an expression product capable of eliciting a prophylactic and/or therapeutic response in a subject, preferably in a human or animal subject), such as for example a (preferably heterologous) peptide, polypeptide or protein, and more particularly antigens and/or non-vaccinogenic prophylactically and/or therapeutically active peptides, polypeptides or proteins. To provide for the expression of said heterologous expression product(s), the microbe may commonly comprise a recombinant nucleic acid encoding the (preferably heterologous) expression product(s). Advantageously, the recombinant nucleic acid may comprise suitable regulatory sequence(s) (e.g., a promoter) operably linked to one or more open reading frames encoding the (preferably heterologous) expression product(s). Hence, also disclosed is a method for reducing or abolishing the colonization capacity of a microbe, wherein said microbe expresses a (preferably heterologous) expression product, said method comprising rendering defective a thymidylate synthase (thyA) gene in said microbe.

A related aspect thus provides the use of thymidylate synthase deficiency in a microbe for reducing or abolishing the colonization capacity of said microbe.

These and further aspects and preferred embodiments of the invention are described in the following sections and in the appended claims.

### BRIEF DESCRIPTION OF FIGURES

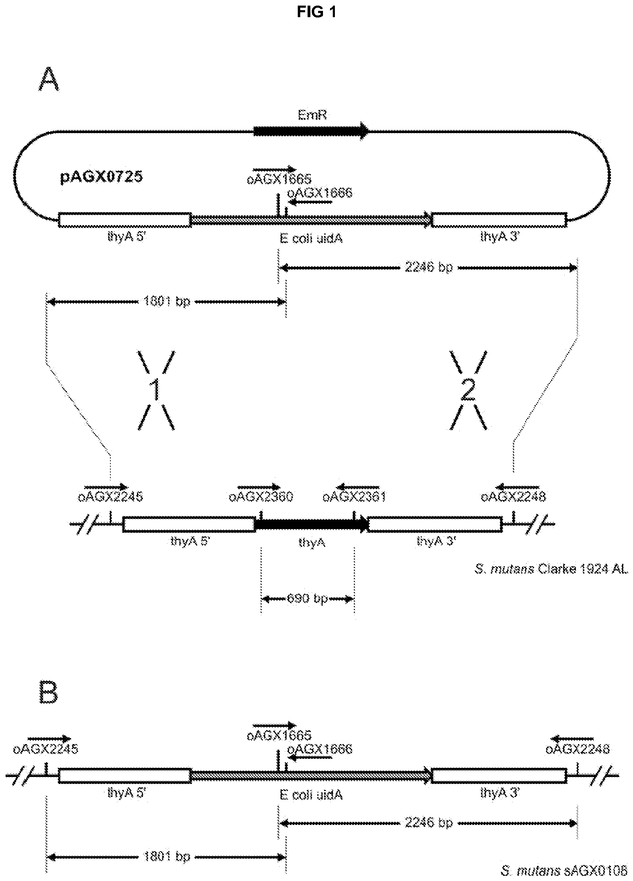

**Figure 1** illustrates generation of thyA- uidA+ *S. mutans* strain sAGX0108. All binding sites of primers oAGX1665, oAGX1666, oAGX2245, oAGX2248, oAGX2360 and oAGX2361are indicated. Note that primers oAGX2245 and oAGX2248 only bind to chromosomal sequences outside of the target areas for recombination and do not bind to pAGX0725. Sizes of PCR products are indicated as bp.
   Panel A: The non-replicative plasmid pAGX0725 was introduced in *S. mutans* strain Clarke 1924 AL through electroporation. Plating on erythromycin and X-gluc containing solid agar plates allowed for the selection of blue erythromycin resistance marker (EmR)+ colonies that have undergone homologous recombination with the bacterial chromosome either upstream (thyA 5'; 1) or downstream (thyA 3'; 2) of thyA. Clones are selected that have undergone recombination at both regions. This leads to the removal of thyA from and the insertion of uidA into the bacterial chromosome. Both homologous recombinations were screened for by PCR using either the primer pair oAGX2245/oAGX1666 (thyA 5') or oAGX1665/oAGX2248 (thyA 3'). Presence or absence of thyA was revealed by PCR using primer pair oAGX2360/oAGX2361.
   Panel B: Final structure of the modified thyA locus of *S. mutans* strain sAGX0108. The presence of uidA in the *S. mutans* sAGX0108 is demonstrated by PCRs using primer pairs oAGX2245/oAGX1666 (showing co-linearity between uidA and the chromosome upstream) and oAGX1665/oAGX2248 (showing co-linearity between uidA and the chromosome downstream). Presence or absence of thyA is revealed by PCR using primer pair oAGX2360/oAGX2361.
   Genomic structures and binding of primers are based on published (*S. mutans* Clarke 1924 AL, assumed to be identical to NCBI Reference Sequence NC_004350.1) or predicted (*S*. *mutans* sAGX0108: in the NCBI Reference Sequence NC_004350.1 the thyA gene was replaced with the *E. coli* uidA gene, GenBank: CP001396.1; complement position 1584343..1586154) DNA sequences.

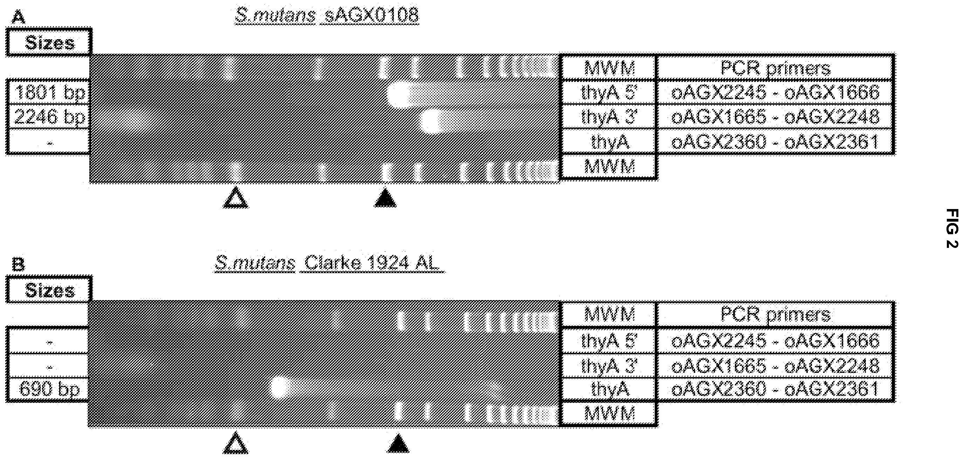

**Figure 2** illustrates PCR analysis of the thyA locus of thyA- *S. mutans* sAGX0108 and its parent thyA+ *S. mutans* Clarke 1924 AL.
   Panel A: PCR reactions performed on DNA extracts from *S. mutans* sAGX0108 using the indicated primers (PCR primers) show the presence of appropriately sized products overlapping both thyA 5' (thyA 5') and thyA 3' (thyA 3') cross over regions while no PCR product corresponding the thyA gene (thyA) can be detected. Primers oAGX1665 and oAGX1666 bind the uidA gene so both PCR reactions that use these primers provide evidence for the presence of the uidA gene.
   Panel B: PCR reactions performed on DNA extracts from *S. mutans* Clarke 1924 AL using the indicated primers show the absence of products overlapping both thyA 5' and thyA 3' cross over regions while an appropriately sized PCR product corresponding to the thyA gene can be detected.
   Sizes indicate the expected size of individual PCR products using the indicated primers and published (*S. mutans* Clarke 1924 AL: assumed to be identical to NCBI Reference Sequence NC_004350.1) or predicted (*S*. *mutans* sAGX0108: in NCBI Reference Sequence NC_004350.1 the thyA gene was replaced with the *E. coli* uidA gene, GenBank: CP001396.1; complement bp position 1584343..1586154) DNA sequences. MWM: Molecular weight markers, contains the following discrete bands (in base pairs): 75, 134, 154, 201, 220, 298, 344, 396, 506, 517 (Δ), 1018, 1636 (▲), 2036, 3054, 4072, 5090, 6108, 7126, 8144, 9162, 10180, 11198, 12216.

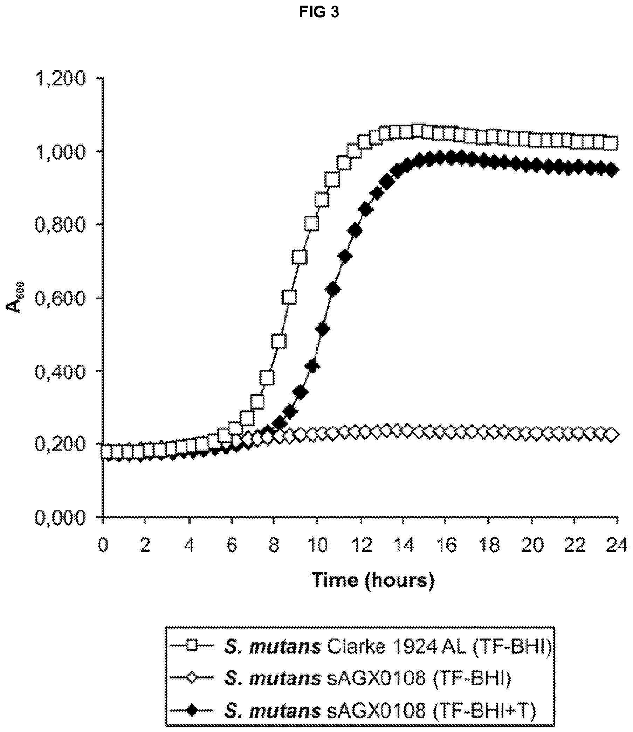

**Figure 3** illustrates growth of WT *S. mutans* Clarke 1924 AL and thyA- *S. mutans* sAGX0108 as monitored by automated measurement of absorbance at 600 nm (A₆₀₀). Cultures were grown in thymidine free Brain Heart Infusion broth (TF-BHI). While *S. mutans* Clarke 1924 AL shows growth to complete saturation, no growth could be observed for *S. mutans* sAGX0108 in the absence of thymidine. The addition of thymidine (+T) to TF-BHI cultures of *S. mutans* sAGX0108 complements its growth deficiency and supports growth to saturation. All data presented are averages of measurements performed on three individual cultures grown in a Bioscreen C MBR automated turbidimeter. Absorbance values at 0 hours reflect A₆₀₀ of TF-BHI.

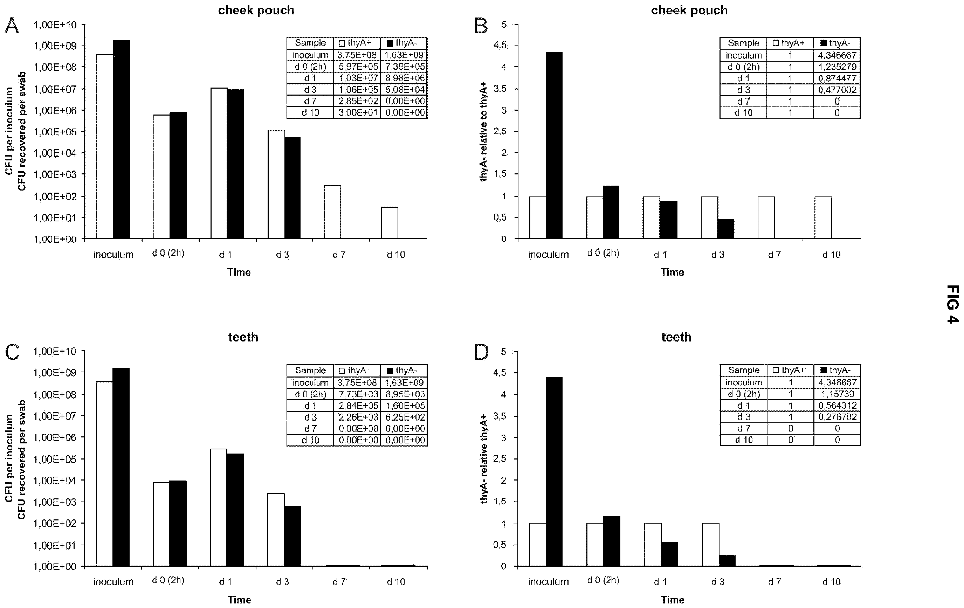

**Figure 4** illustrates relative colonization of thyA+ (*S. mutans* Clarke 1924 AL pILPOL; white bars) and thyA- (*S. mutans* sAGX0108; black bars) *S. mutans* in the oral cavity of hamsters. The entire dental surface of hamsters was cleaned with cotton swabs immediately prior to inoculation with *S*. *mutans.* Saturated overnight cultures of *S. mutans* Clarke 1924 AL pILPOL and *S. mutans* sAGX0108 were concentrated 50x in BAM9T. Of both strains, concentrations were determined and presented as #CFU per inoculum (inoculum). The concentrated suspensions were mixed 50:50 and hamsters were inoculated in the left cheek pouch with 50 µl of the bacterial suspension. Using cotton swabs, samples were taken from the left cheek pouch (cheek) and entire dental surface (teeth) 2 hours (d 0 (2h)), 1 day (d 1), 3 days (d 3), 7 days (d 7) and 10 days (d 10) subsequent to inoculation. 8 hamsters were used in this experiment. Hamsters were used for sampling cheek pouch and dental surface at one time point only. At 2 hours, 1 day and 3 days, samples were taken from 2 hamsters and averages were calculated. At day 7 and 10 samples were taken from 1 hamster. Cotton tips holding the samples were cut off the shaft, submerged in 1 ml of 1xM9 and mixed thoroughly. To determine the concentrations of *S. mutans* Clarke 1924 AL pILPOL and *S. mutans* sAGX0108 present on the swabs, sample suspensions were diluted appropriately in 1xM9 and plated in duplicate on BHI solid agar plates containing erythromycin and BHI solid agar plates containing X-gluc and thymidine respectively. Panel A shows #CFU of both strains in the inoculum (CFU per inoculum) and in samples from the left cheek pouch, taken at the different time points (CFU recovered per swab). Panel B shows the relative presence of thyA- vs thyA+ *S. mutans* (set as 1) in the inoculum and in samples from cheek pouches taken at the different time points. Panel C shows #CFU of both strains in the inoculum (CFU per inoculum) and in samples from the entire dental surface, taken at the different time points (CFU recovered per swab). Panel D shows the relative presence of thyA- vs thyA+ *S. mutans* (set as 1, except when no thyA+ were recovered) in the inoculum and in samples from dental surfaces taken at the different time points. For all panels, exact values are shown in the inserts.

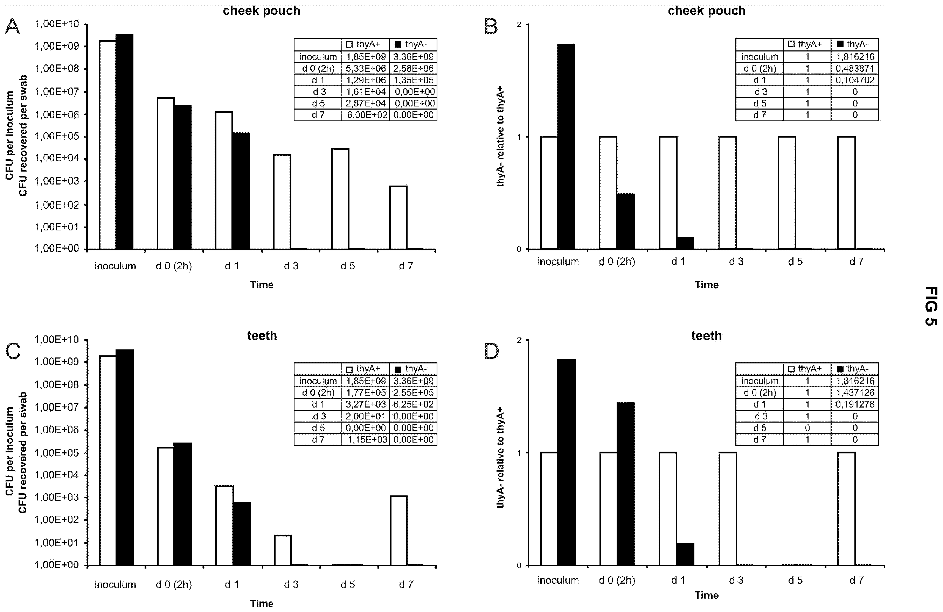

**Figure 5** illustrates relative colonization of thyA+ (*S. mutans* Clarke 1924 AL pILPOL Cm+; white bars) and thyA- (*S. mutans* sAGX0108 Cm+; black bars) *S. mutans* in the oral cavity of hamsters. The entire dental surface of hamstes was cleaned with cotton swabs immediately prior to inoculation with *S. mutans.* Saturated overnight cultures of *S. mutans* Clarke 1924 AL pILPOL Cm+ and *S. mutans* sAGX0108 Cm+ were concentrated 50x in BAM9T. Of both strains, concentrations were determined and presented as #CFU per inoculum (inoculum). The concentrated suspensions were mixed 50:50 and hamsters were inoculated in the left cheek pouch with 50 µl of the bacterial suspension. Using cotton swabs, samples were taken from the left cheek pouch (cheek) and entire dental surface (teeth) 2 hours (d 0 (2h)), 1 day (d 1), 3 days (d 3), 5 days (d 5) and 7 days (d 7) subsequent to inoculation. 8 hamsters were used in this experiment. Hamsters were used for sampling cheek pouch and dental surface at one time point only. At 2 hours, 1 day and 3 days, samples were taken from 2 hamsters and averages were calculated. At day 5 and 7 samples were taken from 1 hamster. Cotton tips holding the samples were cut off the shaft, submerged in 1 ml of 1xM9 and mixed thoroughly. To determine the concentrations of *S. mutans* Clarke 1924 AL pILPOL Cm+ and *S. mutans* sAGX0108 Cm+ present on the swabs, sample suspensions were diluted appropriately in 1xM9 and plated on BHI solid agar plates containing erythromycin and BHI solid agar plates containing X-gluc and thymidine respectively.
   Panel A shows #CFU of both strains in the inoculum (CFU per inoculum) and in samples from the left cheek pouch, taken at the different time points (CFU recovered per swab). Panel B shows the relative presence of thyA- vs thyA+ *S. mutans* (set as 1) in the inoculum and in samples from cheek pouches taken at the different time points. Panel C shows #CFU of both strains in the inoculum (CFU per inoculum) and in samples from the entire dental surface, taken at the different time points (CFU recovered per swab). Panel D shows the relative presence of thyA- vs thyA+ *S. mutans* (set as 1, except when no thyA+ were recovered) in the inoculum and in samples from dental surfaces taken at the different time points. For all panels, exact values are shown in the inserts.

### DETAILED DESCRIPTION OF THE INVENTION

As used herein, the singular forms "a", "an", and "the" include both singular and plural referents unless the context clearly dictates otherwise. By way of example, "a cell" refers to one or more than one cell.

The terms "comprising", "comprises" and "comprised of as used herein are synonymous with "including", "includes" or "containing", "contains", and are inclusive or open-ended and do not exclude additional, non-recited members, elements or method steps. The term also encompasses "consisting of".

The recitation of numerical ranges by endpoints includes all numbers and fractions subsumed within that range, as well as the recited endpoints.

The term "about" as used herein when referring to a measurable value such as a parameter, an amount, a temporal duration, and the like, is meant to encompass variations of and from the specified value, in particular variations of +/-20% or less, preferably +/-10% or less, more preferably +/-5% or less, even more preferably +/-1% or less, and still more preferably +/-0.1% or less of and from the specified value, insofar such variations are appropriate to perform in the disclosed invention. It is to be understood that the value to which the modifier "about" refers is itself also specifically, and preferably, disclosed.

Unless otherwise defined, all terms used in disclosing the invention, including technical and scientific terms, have the meaning as commonly understood by one of ordinary skill in the art to which this invention belongs. By means of further guidance, ensuing definitions are included to better appreciate the teaching of the present invention.

The term "nucleic acid" as used herein means a polymer of any length composed essentially of nucleotides, e.g., deoxyribonucleotides and/or ribonucleotides. Nucleic acids can comprise purine and/or pyrimidine bases and/or other natural (e.g., xanthine, inosine, hypoxanthine), chemically or biochemically modified (e.g., methylated), non-natural, or derivatised nucleotide bases. The backbone of nucleic acids can comprise sugars and phosphate groups, as can typically be found in RNA or DNA, and/or one or more modified or substituted sugars and/or one or more modified or substituted phosphate groups. The term "nucleic acid" further preferably encompasses DNA, RNA and DNA/RNA hybrid molecules, specifically including hnRNA, pre-mRNA, mRNA, cDNA, genomic DNA, amplification products, oligonucleotides, and synthetic (e.g. chemically synthesised) DNA, RNA or DNA/RNA hybrids. A "nucleic acid" can be double-stranded, partly double stranded, or single-stranded. Where single-stranded, the nucleic acid can be the sense strand or the antisense strand. In addition, nucleic acid can be circular or linear.

In a preferred embodiment, a nucleic acid may be DNA or RNA, more preferably DNA.

The term "recombinant nucleic acid" refers generally to a nucleic acid which is comprised of segments joined together using recombinant DNA technology. When a recombinant nucleic replicates in a host organism, the progeny nucleic acids are also encompassed within the term "recombinant nucleic acid".

Standard reference works setting forth the general principles of recombinant DNA technology include Molecular Cloning: A Laboratory Manual, 2nd ed., vol. 1-3, ed. Sambrook et al., Cold Spring Harbor Laboratory Press, Cold Spring Harbor, N.Y., 1989; Current Protocols in Molecular Biology, ed. Ausubel et al., Greene Publishing and Wiley-Interscience, New York, 1992 (with periodic updates); Innis et al., PCR Protocols: A Guide to Methods and Applications, Academic Press: San Diego, 1990. General principles of microbiology are set forth, for example, in Davis, B. D. et al., Microbiology, 3rd edition, Harper & Row, publishers, Philadelphia, Pa. (1980).

Expression of peptides, polypeptides and proteins can be achieved through operably linking nucleic acid sequences or open reading frame(s) (ORFs) encoding said products with regulatory sequences allowing for expression of the nucleic acids or ORFs, e.g., in the microbes and/or strains taught herein. Such expression may be achieved, e.g., under suitable (culture) conditions or upon addition of inducers (e.g., where inducible regulatory sequences are used).

An "operable linkage" is a linkage in which regulatory sequences and sequences sought to be expressed are connected in such a way as to permit said expression. For example, sequences, such as, e.g., a promoter and an ORF, may be said to be operably linked if the nature of the linkage between said sequences does not: (1) result in the introduction of a frame-shift mutation, (2) interfere with the ability of the promoter to direct the transcription of the ORF, (3) interfere with the ability of the ORF to be transcribed from the promoter sequence.

The precise nature of regulatory sequences or elements required for expression may vary between expression environments, but typically include a promoter and a transcription terminator, and optionally an enhancer.

Reference to a "promoter" or "enhancer" is to be taken in its broadest context and includes transcriptional regulatory sequences required for accurate transcription initiation and where applicable accurate spatial and/or temporal control of gene expression or its response to, e.g., internal or external (e.g., exogenous) stimuli. By "promoter" is meant generally a region on a nucleic acid molecule, preferably DNA molecule, to which an RNA polymerase binds and initiates transcription. A promoter is preferably, but not necessarily, positioned upstream, i.e., 5', of the sequence the transcription of which it controls. A promoter may optionally comprise an operator configured to control transcription from the promoter. As used herein, the term "operator" refers to a nucleotide sequence, preferably DNA sequence, which controls the initiation and/or maintenance of transcription of a sequence from a promoter.

The term "microbes" generally refers to microorganisms and particularly refers to bacteria, yeast, fungus. The term "colonizing microbes" refers to those bacteria, yeast and fungi that are capable of residing in the mucosa of the animal or human subject, in particular in the mucosa of the alimentary tract. Hence, the term "colonizing microbes" would be readily understood by a skilled person, in particular to encompass microbes that are able to survive, grow and remain in a given site such as a mucosa for extended periods of time. The colonizing nature or colonization capacity of a microbe may be determined by means available in the art, such as by analyzing biopsy samples or mucus samples in order to determine persistence of microbes in said samples, or taking stool samples to evaluate excretion of microbes.

While having a clear denotation per se, the term "reducing" with reference to the colonization capacity may in particular refer to any qualitative and/or quantitative alteration, change or variation which decreases or diminishes the colonizing character of a microbe. By means of example and not limitation, manifestations of reduced colonizing capacity may include a comparably lower titer of microbe persisting at a given site and/ or a comparably faster clearance of a microbe from a given site, etc.

The term encompasses any extent of such reduction. Where said reduction can be monitored in terms of a quantifiable variable (e.g., the titer of a microbe in a sample from a given site, e.g., at one or more time points) the "reduction" may particularly encompass a decrease in the value of said variable by at least about 10%, e.g., by at least about 20%, preferably by at least about 30%, e.g., by at least about 40%, more preferably by at least about 50%, e.g., by at least about 60%, even more preferably by at least about 70%, e.g., by at least about 80%, still more preferably by at least about 90%, e.g., by at least about 95%, such as by at least about 96%, 97%, 98%, 99% or even by 100%, compared to a reference microbe.

Further, conform its common meaning, the term "abolish" as used herein particularly encompasses removing or eliminating the colonizing capacity of a microbe such that the so-modified microbe would be deemed to have become non-colonizing.

Examples of such colonizing microbes include but are not limited to *Candida sp., Aspergillus sp., Penicillium sp., Saccharomyces sp., Hansenula sp., Kluyveromyces sp. Schizzosaccharomyces sp. Zygosaccharomyces sp., Pichia sp., Monascus sp., Geotrichum sp, Yarrowia sp. Bacteroides sp, Clostridium sp*., *Fusobacterium sp., Eubacterium sp., Ruminococcus sp., Peptococcus sp., Peptostreptococcus sp., Streptococcus sp., Bifidobacterium sp., Escherichia sp.* and *Lactobacillus sp.*

The term *"Bifidobacterium"* or *"Bifidobacterium sp."* generally refers to the genus *Bifidobacterium* and encompasses any taxon (e.g., species, subspecies, strain) classified as belonging to such in the art. By means of example, *Bifidobacterium* or *Bifidobacterium sp.* includes the species *B*. *adolescentis, B*. *angulatum, B. animalis, B*. *asteroides, B. bifidum, B. boum, B. breve, B. catenulatum, B. choerinum, B. coryneforme, B. cuniculi, B. denticolens, B. dentium, B. gallicum, B. gallinarum, B. indicum, B. infantis, B. inopinatum, B. lactis, B. longum, B. magnum, B. merycicum, B. minimum, B. pseudocatenulatum, B. pseudolongum, B. pullorum, B. ruminantium, B. saeculare, B. subtile, B. suis, B. thermacidophilum* and *B*. *thermophilum.*

The term *"Lactobacillus"* or *"Lactobacillus sp."* generally refers to the genus *Lactobacillus* and encompasses any taxon (e.g., species, subspecies, strain) classified as belonging to such in the art. By means of example, *Lactobacillus* or *Lactobacillus sp.* includes the species *L. acetotolerans, L. acidifarinae, L. acidipiscis, L. acidophilus, L. agilis, L. algidus, L. alimentarius, L. amylolyticus, L. amylophilus, L. amylotrophicus, L. amylovorus, L. animalis, L. antri, L. apodemi, L. aviarius, L. bifermentans, L. brevis, L. buchneri, L. camelliae, L. casei, L. catenaformis, L. ceti, L. coleohominis, L. collinoides, L. composti, L. concavus, L. coryniformis, L. crispatus, L. crustorum, L. curvatus, L. delbrueckii, L. delbrueckii subsp. bulgaricus, L. delbrueckii subsp. lactis, L. diolivorans, L. equi, L. equigenerosi, L. farraginis, L. farciminis, L. fermentum, L. fornicalis, L. fructivorans, L. frumenti, L. fuchuensis, L. gallinarum, L. gasseri, L. gastricus, L. ghanensis, L. graminis, L. hammesii, L. hamsteri, L. harbinensis, L. hayakitensis, L. helveticus, L. hilgardii, L. homohiochii, L. iners, L. ingluviei, L. intestinalis, L. jensenii, L. johnsonii, L. kalixensis, L. kefiranofaciens, L. kefiri, L. kimchii, L. kitasatonis, L. kunkeei, L. leichmannii, L. lindneri, L. malefermentans, L. mali, L. manihotivorans, L. mindensis "L. mucosae, L. murinus, L. nagelii, L. namurensis, L. nantensis, L. oligofermentans, L. oris, L. panis, L. pantheris, L. parabrevis, L. parabuchneri, L. paracollinoides, L. parafarraginis, L. parakefiri, L. paralimentarius, L. paraplantarum, L. pentosus, L. perolens, L. plantarum, L. pontis, L. psittaci, L. rennini, L. reuteri, L. rhamnosus, L. rimae, L. rogosae, L. rossiae, L. ruminis, L. saerimneri, L. sakei, L. salivarius, L. sanfranciscensis, L. satsumensis, L. secaliphilus, L. sharpeae, L. siliginis, L. spicheri, L. suebicus, L. hailandensis, L. ultunensis, L. vaccinostercus, L. vaginalis, L. versmoldensis, L. vini, L. vitulinus, L. zeae, L. zymae* and any subspecies and strains thereof. In preferred embodiments of the invention the *Lactobacillus* is *Lactobacillus casei or. Lactobacillus plantarum* or *Lactobacillus rhamnosus.* In further preferred embodiments of the invention the *Lactobacillus* is *Lactobacillus salivarius.*

The term *"Bacteroides"* or *"Bacteroides sp."* generally refers to the genus *Bacteroides* and encompasses any taxon (e.g., species, subspecies, strain) classified as belonging to such in the art. By means of example, *Bacteroides* or *Bacteroides sp.* includes the species *B*. *acidifaciens, B. distasonis, B. gracilis, B. eggerthii, B. fragilis, B. oris, B. ovatus, B. putredinis, B. pyogenes, B. stercoris, B. suis, B. tectus, B. thetaiotaomicron, B. uniformis, B. vulgatus,* and any subspecies and strains thereof. In preferred embodiments of the invention the *Bacteroides* is *Bacteroides ovatus.*

The term *"Streptococcus"* or *"Streptococcus* sp." generally refers to the genus *Streptococcus* and encompasses any taxon (e.g., species, subspecies, strain) classified as belonging to such in the art. By means of example, *Streptococcus* or *Streptococcus* sp. includes the species *S. agalactiae, S. anginosus, S. bovis, S. canis, S. equi, S. iniae, S. mitis, S. mutans, S. oralis, S. parasanguinis, S. peroris, S. pneumoniae, S. pyogenes, S. ratti, S. salivarius, S. salivarius* ssp. *thermophilus, S. sanguinis, S. sobrinus, S. suis, S. uberis, S. vestibularis, S. viridans,* and any subspecies and strains thereof. Preferably the *Streptococcus* is *Streptococcus mutans.*

The term "thymidylate synthase" is common in the art and generally refers to the enzyme EC 2.1.1.45, also known under the synonymous denotations dTMP synthase, thymidylate synthetase, methylenetetrahydrofolate:dUMP C-methyltransferase and TMP synthetase. Thymidylate synthase activity in particular involves catalyzing reaction of 5,10-methylenetetrahydrofolate + dUMP to dihydrofolate + dTMP. A thymidylate synthase may be generally encoded by and expressed from a thymidylate synthase gene, abbreviated as "thyA" gene. By means of example, the term "thymidylate synthase (*thyA*) gene of *Lactobacillus"* denotes a gene encoding the said enzyme in a *Lactobacillus.* The sequence of the *thyA* gene from several *Lactobacillus* taxons has been described, such as, e.g., from *Lactobacillus delbrueckii* subsp. *Bulgaricus* (Sasaki et al., 2004 (Appl Environ Microbiol 70(3): 1858-64), *Lactobacillus casei* (Genbank Gene ID: 4419806). A skilled person is capable of identifying and isolating *thyA* gene homologues from further taxons of *Lactobacillus.*

In the present disclosure, the thymidylate synthase (thyA) gene may be rendered defective or inactive in the microbe. Rendering a thyA gene defective or inactive may in particular involve modifying or altering the thyA gene such that the thymidylate synthase activity expressed in the microbe from said thyA gene is reduced or preferably abolished.

Whereas a thyA gene defect as intended herein may lead to any extent of reduction of thymidylate synthase activity expressed in the microbe from said thyA gene, particularly useful may be a substantial reduction of the thymidylate synthase activity in the microbe, such as for example a reduction by at least about 30%, e.g., by at least about 40%, preferably by at least about 50%, e.g., by at least about 60%, more preferably by at least about 70%, e.g., by at least about 80%, even more preferably by at least about 90%, e.g., by at least about 95%, such as by at least about 96%, 97%, 98%, 99% or even by 100%, compared to a non-defective (active) thyA gene. Preferably, the thyA gene defect may result in no thymidylate synthase activity being produced from said thyA gene, i.e., in abolishment of said activity. The activity of thymidylate synthase may be measured, without limitation, by established enzymatic assays or by assessing the degree of thymine and/or thymidine auxotrophy of a microbe.

A thyA gene in a microbe may be rendered defective, for example, by suitably altering the regulatory sequences controlling the thyA expression and/or by suitably altering the sequences (ORF) coding for the thymidylate synthase.

Preferably, the alteration may involve gene disruption. The term "gene disruption" may commonly encompass genetic alterations that reduce or preferably abolish the encoded gene product activity, and in particular gene disruption, as used throughout this specification, includes disruption by insertion of a DNA fragment, disruption by deletion of the gene, or a part thereof, as well as exchange of the gene or a part thereof by another DNA fragment, and said disruption is induced by recombinant DNA techniques, and not by spontaneous mutation. Preferably, disruption is the exchange of the gene, or a part thereof, by another functional gene.

In an embodiment, the bacterium may possess inherent prophylactic and/or therapeutic traits, such as for example, the bacterium may be deemed a probiotic. The term "probiotic" is know in the art and may particularly encompass microbial food or feed supplements whose primary aim is to maintain and/or improve the health and/or well-being of a subject such as a human or animal. Beneficial effects of probiotics may in particular be due to improving mucosal microbial balance, such as microbial balance in the alimentary tract or parts thereof.

In a further embodiment, the bacterium may express a heterologous expression product. The term "heterologous" when referring to the relationship between an expression product and a microbe means that said expression product is not normally expressed by said microbe in nature. In particular, expression of said expression product by the microbe may be created using recombinant DNA techniques, more in particular through introducing to the microbe a recombinant nucleic acid encoding and effecting the expression of said expression product in said microbe.

As used herein, the term "antigen" generally refers to a substance foreign to a body (esp. to a body of a human or animal subject whereto the antigen is to be administered) that evokes an immune response, including humoral immunity and/or cellular immunity response, and that is capable of binding with a product, e.g., an antibody or a T cell, of the immune response. Hence, in a preferred example, an antigen requires a functioning immune system of a subject to which it is administered to elicit a physiological response from such a subject.

An antigen according to the invention may be derived from any polypeptide to which an immune response in a human or animal subject would be therapeutically useful, e.g., from a pathogen, e.g., from a viral, prokaryotic (e.g., bacterial) or eukaryotic pathogen, from a non-physiological protein (e.g., a protein derived from cancer tissue), from allergen (e.g., for eliciting immune tolerance), etc.

Hence, in a preferred embodiment an antigen is capable of eliciting an immune tolerance response in a subject such as a human or animal.

The term "alimentary canal" is known in the art and may particularly encompass the mouth, oesophagus, stomach, small intestine (including inter alia Duodenum, Jejunum and Ileum) and large intestine (colon) rectum and anus. The phrase "mucosa of the alimentary canal" may refer to mucosa of any one or more or all sites of the alimentary canal, as may be apparent from the context.

The term "a non-vaccinogenic therapeutically active polypeptide" refers generally to a polypeptide that, in a human or animal subject to which it is administered, does not elicit an immune response against itself and is able to achieve a therapeutic effect. Hence, the therapeutic effect of such a polypeptide would be expected to be directly linked to its own natural biological function whereby it can achieve particular effects in a body of a subject; rather than producing a therapeutic effect by acting as an immunogenic and/or immunoprotective antigen in the subject. Hence, the non-vaccinogenic therapeutically active polypeptide should be biologically active in its expressed form or, at least, must be converted into the biologically active form once released from the expressing host cell. Preferably, such biologically active form of the said polypeptide may display a secondary and preferably also tertiary conformation which is the same or closely analogous to its native configuration.

Preferably, the non-vaccinogenic therapeutically active polypeptide is also non-toxic and non-pathogenic.

In a preferred embodiment, the non-vaccinogenic therapeutically active polypeptide may be derived from human or animal, and may preferably correspond to the same taxon as the human or animal subject to which it is to be administered.

Non-limiting examples of suitable non-vaccinogenic therapeutically active polypeptides include ones which are capable of functioning locally or systemically, e.g., is a polypeptide capable of exerting endocrine activities affecting local or whole-body metabolism and/or the biologically active polypeptide(s) is/are one(s) which is/are capable of the regulation of the activities of cells belonging to the immunohaemopoeitic system and/or the one or more biologically active polypeptides is/are one(s) which is/are capable of affecting the viability, growth and differentiation of a variety of normal or neoplastic cells in the body or affecting the immune regulation or induction of acute phase inflammatory responses to injury and infection and/or the one or more biologically active polypeptides is/are one(s) which is/are capable of enhancing or inducing resistance to infection of cells and tissues mediated by chemokines acting on their target cell receptors, or the proliferation of epithelial cells or the promotion of wound healing and/or the one or more biologically active polypeptides modulates the expression or production of substances by cells in the body.

Specific examples of such polypeptides include, without limitation, insulin, growth hormone, prolactin, calcitonin, luteinising hormone, parathyroid hormone, somatostatin, thyroid stimulating hormone, vasoactive intestinal polypeptide, cytokines (including but not limited to interleukins IL-2, IL-3, IL-4, IL-5, IL-6, IL-7, IL-9, IL-10,IL-11, IL-12, IL-13, IL-17 any of IL-14 to IL-35), GM-CSF, M-CSF, SCF, IFNs, EPO,G-CSF, LIF, OSM, CNTF, GH, PRL, the TNF family of cytokines, e.g., TNFα, TNPβ, CD40, CD27 or FAS ligands, the IL-1 family of cytokines, the fibroblast growth factor family, the platelet derived growth factors, transforming growth factors and nerve growth factors, the epidermal growth factor family of cytokines, the insulin related cytokines, etc. Alternatively, the therapeutically active polypeptide can be a receptor or antagonist for the therapeutically active polypeptides as defined above. Further specific examples of such suitable polypeptides are listed, e.g., in WO 96/11277, page 14, lines 1-30; in WO 97/14806, page 12, line 1 through page 13, line 27; or US 5,559,007, col. 8, line 31 through col. 9, line 9. In an embodiment, said non-vaccinogenic therapeutically active polypeptide may be hIL-10, GLP-2, GLP-1, TFF or hPYY.

Accordingly, in an embodiment the bacterium may comprise a recombinant nucleic acid which encodes an antigen and/or a non-vaccinogenic prophylactically and/or therapeutically active polypeptide, wherein the said antigen is capable of eliciting an immune response, preferably protective immune response or immune tolerance response, in a human or animal subject, and/or the said non-vaccinogenic therapeutically active polypeptide is capable of producing a prophylactic and/or therapeutic effect in a human or animal subject.

WO 97/14806 further specifically discloses co-expression of antigens with immune response stimulatory molecules, such as, e.g., interleukins, e.g., IL-2 or IL-6, by bacteria. Accordingly, such co-expression of two or more antigens and/or non-vaccinogenic prophylactically and/or therapeutically active polypeptides in a microbe as taught herein is also contemplated.

In a further preferred embodiment, the open reading frame encoding a (preferably heterologous) expression product further comprises a sequence encoding a secretion signal in phase with a polypeptide encoded by the ORF. This advantageously allows for secretion of the expressed polypeptide from the host cell and thereby may facilitate, e.g., isolation or delivery.

Typically, a secretion signal sequence represents an about 16 to about 35 amino acid segment, usually containing hydrophobic amino acids that become embedded in the lipid bilayer membrane, and thereby allow for the secretion of an accompanying protein or peptide sequence from the host cell, and which usually is cleaved from that protein or peptide. Preferably, the secretion signal sequence may be so-active in a host cell intended for use with the nucleic acid comprising the said signal sequence, e.g., a bacterial host cell, preferably a lactic acid bacterium, more preferably *Lactobacillus.*

Secretion signal sequences active in suitable host cells are known in the art; exemplary *Lactobacillus* signal sequences include those of usp45 (see, US 5,559,007), usp45N4 (see WO 2008/084115) and others, see, e.g., (Perez-Martinez et al., 1992 (Mol Gen Genet 234(3): 401-11); (Sibakov et al., 1991 (Appl Environ Microbiol 57(2): 341-8). Preferably, the signal sequence is located between the promoter sequence and the ORF, i.e. the signal sequence is located 3' from the promoter sequence and precedes the ORF of the polypeptide of interest. In a preferred embodiment, the signal sequence encodes the amino acid sequence MKKKIISAIL MSTVILSAAA PLSGVYA (usp45) (SEQ ID NO: 9).

The recombinant nucleic acid may comprise a promoter, being a native promoter from a microbe (e.g., *Lactobacillus, Streptococcus, Bacteroides,* or *Lactococcus* species) or a functional variant or functional fragment thereof, operably linked to one or more open reading frames encoding a (preferably heterologous) expression product.

The promoter may be chosen from the group comprising or consisting of the native promoters of genes of *Lactococcus* for (see, e.g., WO 2008/084115): 1) DNA-directed RNA polymerase, beta' subunit / 160 kD subunit (*rpoC*), 2) DNA-directed RNA polymerase, beta subunit / 140 kD subunit (*rpoB*), 3) non-heme iron-binding ferritin (*dpsA*), 4) pyruvate kinase (*pyk*), 5) glutamyl-tRNA synthetases (*gltX*), 6) phosphopyruvate hydratase (*eno*), 7) glutamine synthetase (*glnA*) 8) glutamine synthetase repressor (*glnR*), 9) dipeptidase PepV (*pepV*), 10) F0F1-type ATP synthase beta subunit (ATP synthase F1 beta subunit) (*atpD*), 11) 3-phosphoglycerate kinase (*pgk*), 12) glyceraldehyde-3-phosphate dehydrogenase (*gapB*), 13) acetate kinase (*ackA*), 14) 3-oxoacyl-(acyl-carrier-protein) synthase II (*fabF*), 15) 3-ketoacyl-(acyl-carrier-protein) reductase (*fabG or fabG1*), 16) DNA-directed RNA polymerase, alpha subunit / 40 kD subunit (*rpoA*), 17) Proline dipeptidase (*pepQ*), 18) fructose-bisphosphate aldolase (*fbaA*), 19) ribosomal protein S4 (*rpsD*), 20) superoxide dismutase (*sodA*), 21) ribosomal protein S12 (*rpsL*) and ribosomal protein S7 (*rpsG*), 22) ribosomal protein L18 (*rplR*) and ribosomal protein S5 (*rpsE*) and ribosomal protein L30/L7E (*rpmD*), 23) S-ribosylhomocysteinase (*luxS*)*,* 24) ribosomal protein L19 (*rplS*), 25) ribosomal protein S11 (*rpsK or infA*), 26) ribosomal protein L10 (*rplJ*), 27) ribosomal protein L7/L12 (*rplL*), 28) HU-like DNA-binding protein (*hllA*), 29) 50S ribosomal protein L28 (*rpmB*), 30) phosphotransferase system llB component (*ptcB*), 31) F0F1-type ATP synthase alpha subunit (*atpA*), 32) multiple sugar-binding transport ATP-binding protein (*msmK*), 33) pyruvate dehydrogenase E1 component alpha subunit (*pdhA*), 34) cell division protein (*diflVA or ftsA*), 35) UDP-galactopyranose mutase (*glf1*), 36) glutamyl aminopeptidase (*pepA*), 37) predicted dehydrogenase related protein (*llmg_0272*), 38) ribosomal protein S2 (*rpsB*), 39) translation initiation factor 3 (IF-3) (*infC*), 40) ribosomal protein L4 (*rplD*) and ribosomal protein L23 (*rplW*) and ribosomal protein L2 (*rplB*), 41) Phenylalanyl-tRNA synthetase beta chain (*pheT*), 42) transcription elongation factor GreA (*greA*), 43) ATP-dependent Clp protease proteolytic subunit (*clpP*), 44) ribosomal protein L15 (*rplO*), 45) ribosomal protein L11 (*rplK*), 46) ribosomal protein S8 (*rpsH*), 47) ribosomal protein L21 (*rplU*), 48) ribosomal protein S13 (*rpsM*), 49) ribosomal protein S19 (*rpsS*) and ribosomal protein L22 (*rplV*) and ribosomal protein L16 (*rplP*) and ribosomal protein L14 (*rplN*), 50) ribosomal protein S10 (*rpsJ*), 51) co-chaperonin GroES (Hsp10) (*groES*), 52) ribosomal protein L24 (*rplX*) and 53) putative holiday junction resolvase (*llmg_0151*) and functional variants and functional fragments of the said native promoters.

The promoter may be 28) bacterial nucleoid DNA-binding protein / HU-like DNA-binding protein (*hlla* or *hup*), even more preferably, said promoter may be the PhllA promoter.

The promoter may be 3) non-heme iron-binding ferritin (*dpsA* or *LACR_2311*), promoter 9) dipeptidase PepV (*pepV* or *LACR_0908*), or promoter 20) superoxide dismutase (*sodA* or *LACR_0458*), respectively, even more preferably, said promoter may be the PdpsA, PpepV or PsodA promoter.

In all embodiments of the invention, the promoter in the recombinant nucleic acid is the native promoter of a *Lactococcus* gene selected from the group consisting of: glyceraldehyde-3-phosphate dehydrogenase (*gapB*), non-heme iron-binding ferritin (*dpsA*), pyruvate kinase (*pyk*), phosphopyruvate hydratase (*eno*), dipeptidase PepV (*pepV*), superoxide dismutase (*sodA*), ribosomal protein L19 (*rplS*), HU-like DNA binding protein (*hllA*), and 50S ribosomal protein L28 (*rpmB*).

In embodiments, the recombinant nucleic acid may comprise:
(a) PdpsA, usp45 and hIL-10; PdpsA, usp45N4 and hIL-10;
   PpepV, usp45 and hIL-10; PpepV, usp45N4 and hIL-10;
   PsodA, usp45 and hIL-10; PsodA, usp45N4 and hIL-10;
   PhllA, usp45 and hIL-10; PhllA, usp45N4 and hIL-10;
(b) PdpsA, usp45N4 and hTFF1; PdpsA, usp45 and hTFF1;
   PpepV, usp45N4 and hTFF1; PpepV, usp45 and hTFF1;
   PsodA, usp45N4 and hTFF1; PsodA, usp45 and hTFF1;
   PhllA, usp45N4 and hTFF1; PhllA, usp45 and hTFF1;
(c) PdpsA, usp45N4 and hTFF3; PdpsA, usp45 and hTFF3;
   PpepV, usp45N4 and hTFF3; PpepV, usp45 and hTFF3;
   PsodA, usp45N4 and hTFF3; PsodA, usp45 and hTFF3;
   PhllA, usp45N4 and hTFF3; PhllA, usp45 and hTFF3;
(d) PdpsA, usp45N4 and hPYY; PdpsA, usp45 and hPYY;
   PpepV, usp45N4 and hPYY; PpepV, usp45 and hPYY;
   PsodA, usp45N4 and hPYY; PsodA, usp45 and hPYY;
   PhllA, usp45N4 and hPYY; PhllA, usp45 and hPYY; PhllA, usp45 and hPYY G9 (3-36);
(e) PdpsA, usp45N4 and GLP-1; PdpsA, usp45 and GLP-1;
   PpepV, usp45N4 and GLP-1; PpepV, usp45 and GLP-1;
   PsodA, usp45N4 and GLP-1; PsodA, usp45 and GLP-1;
   PhllA, usp45N4 and GLP-1; PhllA, usp45 and GLP-1;
(f) PdpsA, usp45N4 and GLP-2; PdpsA, usp45 and GLP-2;
   PpepV, usp45N4 and GLP-2; PpepV, usp45 and GLP-2;
   PsodA, usp45N4 and GLP-2; PsodA, usp45 and GLP-2;
   PhllA, usp45N4 and GLP-2; or PhllA, usp45 and GLP-2.

As described herein, the promoter may in the alternative be chosen from the group comprising or consisting of the native promoters of genes of *Lactobacillus,* preferably but without limitation of *Lactobacillus rhamnosus,* for 1) ribosomal protein S14 and ribosomal protein S17 and ribosomal protein L15 and ribosomal protein S3 (*rpsJ*), 2) nucleoid DNA-binding protein (*dnabp*), 3) ribosomal protein S21 (*rpS21*)*,* 4) 50S ribosomal protein L19 (*rplS*), 5) 50S ribosomal protein L17 (*rnap40*), 6) 50S ribosomal protein L13 (*rplM*), 7) phosphoglycerate mutase 1 (*pgm1*), 8) ribosomal protein S4 (*rpS4*), 9) glyceraldehyde-3-phosphate dehydrogenase (*cggr*), and functional variants and functional fragments of the said native promoters. Said promoters can ensure particularly strong expression of molecules of interest. Said promoters may find particular use in expression of molecules of interest in *Lactobacillus* sp. such as for example in *L. plantarum, L. acidophilus, L. rhamnosus, L. salivarius* or *L. casei,* and any subspecies and strains thereof.

As described herein, the The promoter may in the alternative be chosen from the group comprising or consisting of the native promoters of genes of *Streptococcus,* preferably but without limitation of *Streptococcus mutans,* for 1) 30S ribosomal protein S10, 2) 50S ribosomal protein L27, 3) 30S ribosomal protein S15, 4) 30S ribosomal protein S16, 5) 50S ribosomal protein L19, 6) 30S ribosomal protein S8, 7) 50S ribosomal protein L18 and 30S ribosomal protein S5, 8) 30S ribosomal protein S9, 9) 50S ribosomal protein L17, 10) 30S ribosomal protein S13, 11) 30S ribosomal protein S7, 12) 50S ribosomal protein L15, 13) 30S ribosomal protein S4, 14) 50S ribosomal protein L6, 15) 30S ribosomal protein S3 and 50S ribosomal protein L3, 16) phosphoglyceromutase, and functional variants and functional fragments of the said native promoters. Said promoters can ensure particularly strong expression of molecules of interest. Said promoters may find particular use in expression of molecules of interest in *Streptococcus* sp. such as for example in *S. mutans,* and any subspecies and strains thereof. By means of example, the above promoters may be chosen from the group comprising or consisting of nucleic acids set forth in Tables 1 and Table 2 below, and functional variants and functional fragments of the said native promoters. In said tables, The Gene ID numbers uniquely identify the said genes in the "Entrez Gene" database of NCBI (www.ncbi.nlm.nih.gov/entrez/query.fcqi?db=:gene) as described in Maglott et al. 2005. (Entrez Gene: gene-centered information at NCBI. Nucleic Acids Res. 33: D54-D58). The NCBI Reference Sequence accession numbers in said Tables provide particular nucleic acid sequence information. Tables 1 and 2 further identify regions of the nucleic acids which may be considered as respective promoter regions.

**Table 1. Select genes and promoter regions identified in Lactobacillus rhamnosus (particularly L. rhamnosus HN001).**

| **Gl-number** | **Gene / Protein name** | **Abbrev.** | **Locus** | **Gene location in NCBI Reference Sequence** | **Promoter region** |
|---|---|---|---|---|---|
| 199598845 | ribosomal protein S14* | rpsJ | LRH_ 03200 | NZ_ ABWJ01000023.1:25789..25974 | 19083-19412 |
| 199598701 | nucleoid DNA-binding protein | *dnabp* | LRH_04048 | comptement(NZ_ABWJ010000200.1.15543..15818) | c(15819-16051) |
| 199599325 | ribosomal protein S21 | *rpS21* | LRH_ 09358 | complement(NZ_ ABWJ01000036.1:5953..6129) | c(6130-6401) |
| 199598841 | ribosomal protein S17* | rpsJ | LRH_ 03180 | NZ_ ABWJ01000023.1:24203..24466 | 19083-19412 |
| 199597121 | 50S ribosomal protein L19 | rpJS | LRH_ 07296 | complement(NZ_ABWJG1000002.1:62543..62890) | c(62891-63094) |
| 199598859 | 50S ribosomal protein L17 | *map40* | LRH_03270 | NZ_ABWJ01000023.1:33024..33404 | 31990-32063 |
| 199598851 | ribosomal protein L15* | rpsJ | LRH_ 03230 | NZ_ ABWJ01000023.1:28112..28552 | 19083-19412 |
| 199596872 | 50S ribosomal protein L13 | rplM | LRH_03335 | NZ_ ABWJ01000023.1:42964..43410 | 42698-42963 |
| 199597445 | phosphoglycerate mutase 1 | *pgm1* | LRH_ 13389 | NZ_ ABWJ01000004.1:98387..99076 | 98118-98386 |
| 199598838 | ribosomal protein S3* | rpsJ | LRH_ 03165 | NZ_ABWJ01000023.1:22898..23580 | 19083-19412 |
| 199597503 | ribosomal protein S4 | *rpS4* | LRH_ 06231 | NZ_ ABWJ01000005.1:20167..2778 | 19992-20166 |
| 199597272 | glyceraklehyde-3-phosphate dehydrogenase | *cgpr* | LRH_ 05289 | NZ_ABWJ01000003.1:50087..51109 | 48208-49012 |

| | | | | | |
|---|---|---|---|---|---|
| * identical operon | | | | | |

**Table 2. Select genes and promoter regions identified in Streptococcus mutans (particularly S. mutans UA159).**

| **Gl-number** | **Gene / Protein name** | **Locus** | **Gene location in NCBI Reference Sequence NC_004350 (version 1)** | **Promoter region** |
|---|---|---|---|---|
| 1350920 | 30S ribosomal protein S10 | SMU.2026c | complement(1893020..1893130) | c1893131-1893647 |
| 24379304 | 50S ribosomal protein L27 | SMU.849 | 797718..798011 | 796862-797035 |
| 24378669 | 30S ribosomal protein S15 | SMU.154 | 156212..156481 | 155470-156212 |
| 24378669 | 30S ribosomal protein S16 | SMU.865 | 814687..814962 | 814285-814686 |
| 24379704 | 50S ribosomal protein L19 | SMU-1288 | complement(1214632..1214979) | c1214980-1215169 |
| 24380355 | 30S ribosomal protein S8 | SMU.2012 | complement(1881696..1888094) | c1888085-1888350 |
| 24380353 | 50S ribosomal protein L18* | SMU.2010 | complement(1886301..1886657) | c1886658-1886746 |
| 24378685 | 30S ribosomal protein S9 | SMU-170 | 170269..170661 | 169406-169798 |
| 24380352 | 30S ribosomal protein S5* | SMU.2009 | complement(1885788..1886282) | c1888658-1886746 |
| 15902260 | 50S ribosomal protein L17 | SMU.2000 | complement(1880033..1880419) | c1881377-1881421 |
| 24380345 | 30S ribosomal protein S13 | SMU.2003 | complement(1881823..1882188) | c1882569-1882686 |
| 24378855 | 30S ribosomal protein S7 | SMU.358 | 334526.334996 | 334997-335164 |
| 24380350 | 50S ribosomal protein L15 | SMU.2007 | complement(1884859..1885299) | c1885300-1885591 |
| 24380465 | 30S ribosomal protein S4 | SMU.2135c | complement(1939236..1999847) | c1999848-1999950 |
| 24380354 | 50S ribosomal protein L6 | SMU.2011 | complement(1886747..1887283) | c1887284-1881695 |
| 161486819 | 30S ribosomal protein S3** | SMU.2021 | complement(1890900..1891553) | c1892786-1893019 |
| 24379074 | phosphoglyceromutase | SMU.74 | 74927..75637 | 74855-74926 |
| 24380367 | 50S ribosomal protein L3** | SMU.2025 | complement(1892159..1892785) | c1892786-1893019 |

| | | | | |
|---|---|---|---|---|
| *identical operon **identical operon | | | | |

The recombinant nucleic acid as taught herein, such as recombinant nucleic acid encoding the (preferably heterologous) expression product, may be comprised in a vector.

As used herein, "vector" refers to a nucleic acid molecule, typically DNA, to which nucleic acid fragments may be inserted and cloned, *i.e.,* propagated. Hence, a vector will typically contain one or more unique restriction sites, and may be capable of autonomous replication in a defined host or vehicle organism such that the cloned sequence is reproducible. Vectors may include, without limitation, plasmids, phagemids, bacteriophages, bacteriophage-derived vectors, PAC, BAC, linear nucleic acids, e.g., linear DNA, etc., as appropriate (see, e.g., (Sambrook et al., Molecular Cloning: A Laboratory Manual, 2nd ed., vol. 1-3, ed. Cold Spring Harbor Laboratory Press, Cold Spring Harbor, N.Y., 1989); (Ausubel et al., Current Protocols in Molecular Biology, ed. Ausubel et al., Greene Publishing and Wiley-Interscience, New York, 1992).

Factors of importance in selecting a particular vector, e.g., a plasmid, include *inter alia:* the ease with which recipient cells that contain the vector may be recognized and selected from those recipient cells which do not contain the vector; the number of copies of the vector which are desired in a particular host; and whether it is desirable to be able to "shuttle" the vector between host cells of different species. Preferred prokaryotic vectors include plasmids such as those capable of replication in *E. coli* (such as, for example, pBR322, ColE1, pSC101, pUC19, etc.). Such plasmids are describe in, e.g., (Sambrook et al., Molecular Cloning: A Laboratory Manual, 2nd ed., vol. 1-3, ed. Cold Spring Harbor Laboratory Press, Cold Spring Harbor, N.Y., 1989); (Ausubel et al., Current Protocols in Molecular Biology, ed. Ausubel et al., Greene Publishing and Wiley-Interscience, New York, 1992). Particularly preferred vectors may be those able to replicate in *E. coli* (or other Gram negative bacteria) as well as in another host cell of interest, such as in a Gram positive bacterium, a lactic acid bacterium, preferably *Lactobacillus.* Other preferred vectors may be those able to replicate and/or shuttle between one or more Gram positive bacteria but not in Gram negative bacteria. In a preferred embodiment, the vector is pT1NX as described by (Steidler et al., 1998 (Infect Immun 66(7): 3183-9) "Mucosal delivery of murine interleukin-2 (IL-2) and IL-6 by recombinant strains of Lactococcus lactis coexpressing antigen and cytokine".

In a related aspect, the invention provides a method for delivery of a (preferably heterologous) expression product, such as polypeptide encoded by the one or more open reading frames comprised within the recombinant nucleic acid of the invention to human or animal in need thereof, comprising administering to the said human or animal a therapeutically effective amount of host cells (strain, microbe) transformed with the said nucleic acid and/or vector of the invention.

The animal may preferably be a mammal, such as, e.g., domestic animals, farm animals, zoo animals, sport animals, pet and experimental animals such as dogs, cats, guinea pigs, rabbits, rats, mice, horses, cattle, cows; primates such as apes, monkeys, orang-utans, and chimpanzees; canids such as dogs and wolves; felids such as cats, lions, and tigers; equids such as horses, donkeys, and zebras; food animals such as cows, pigs, and sheep; ungulates such as deer and giraffes; rodents such as mice, rats, hamsters and guinea pigs; and so on. Generally, the term "subject" or "patient" may be used interchangeably and particularly refer to animals, preferably warm-blooded animals, more preferably vertebrates, even more preferably mammals, still more preferably primates, and specifically includes human patients and non-human animals, mammals and primates. Preferred patients may be human subjects.

As used herein, the terms "treat" or "treatment" refer to both therapeutic treatment and prophylactic or preventative measures, wherein the object is to prevent or slow down (lessen) an undesired physiological change or disorder. A "human or animal in need of treatment" includes ones that would benefit from treatment of a given condition.

The term "therapeutically effective amount" refers to an amount of a therapeutic substance or composition effective to treat a disease or disorder in a subject, e.g., human or animal, i.e., to obtain a desired local or systemic effect and performance. By means of example, a therapeutically effective amount of bacteria may comprise at least 1 bacterium, or at least 10 bacteria, or at least 10² bacteria, or at least 10³ bacteria, or at least 10⁴ bacteria, or at least 10⁵ bacteria, or at least 10⁶ bacteria, or at least 10⁷ bacteria, or at least 10⁸ bacteria, or at least 10⁹, or at least 10¹⁰, or at least 10¹¹, or at least 10¹², or at least 10¹³, or at least 10¹⁴, or at least 10¹⁵, or more host cells, e.g., bacteria, e.g., in a single or repeated dose.

The host cells (strain, microbe) of the present invention may be administered alone or in combination with one or more active compounds. The latter can be administered before, after or simultaneously with the administration of the said host cells.

A number of prior art disclosures on the delivery of antigens and/or therapeutically active polypeptides exist, and it shall be appreciated that such disclosures may be further advantageously modified with the reduced-colonizing or non-colonizing strains of microbes as taught herein. By means of example and not limitation, bacterial delivery of trefoil peptides may be used to treat diseases of the alimentary canal (see, e.g., WO 01/02570), delivery of interleukins in particular IL-10 for treating colitis (e.g., see WO 00/23471), delivery of antigens as vaccines (e.g., WO 97/14806), delivery of GLP-2 and related analogs may be used to treat short bowel disease, Crohn's disease, osteoporosis and as adjuvant therapy during cancer chemotherapy, etc. Further therapeutic applications are envisioned using the promoters and host cells (strain, microbe) of the invention.

Further non-limiting examples of the types of diseases treatable in humans or animals by delivery of therapeutic polypeptides according to the invention include, but are not limited to, e.g., inflammatory bowel diseases including Crohn's disease and ulcerative colitis (treatable with, e.g., IL-Ira or IL-10 or trefoil peptides); autoimmune diseases, including but not limited to psoriasis, rheumatoid arthritis, lupus erythematosus (treatable with, e.g., IL-Ira orlL-10); neurological disorders including, but not limited to Alzheimer's disease, Parkinson's disease and amyotrophic lateral sclerosis (treatable with, e.g., brain devated neurotropic factor and ciliary neurotropic factor); cancer (treatable with, e.g., IL-1, colony stimulating factors or interferon- W); osteoporosis (treatable with, e.g., transforming growth factorf3); diabetes (treatable with, e.g., insulin); cardiovascular disease (treatable with, e.g., tissue plasminogen activator); atherosclerosis (treatable with, e.g., cytokines and cytokine antagonists); hemophilia (treatable with, e.g., clotting factors); degenerative liver disease (treatable with, e.g., hepatocyte growth factor or interferon a); pulmonary diseases such as cystic fibrosis (treatable with, e.g., alpha antitrypsin); obesity; pathogen infections, e.g., viral or bacterial infections (treatable with any number of the above-mentioned compositions or antigens); etc.

The skilled reader shall appreciate that the herein specifically recited diseases are only exemplary and their recitation is in no way intended to confine the use of the reagents provided by the invention, e.g., the reduced-colonizing or non-colonizing strains of microbes of the invention, to these particular diseases. Instead, a skilled reader understands that the reagents of the invention can be used to express in principle any expression products, preferably polypeptides, of interest, which may be of therapeutic relevance in not only the recited ones but also in various further diseases or conditions of humans and animals. Consequently, once a suitable expression product, preferably a polypeptide, e.g., an antigen and/or a non-vaccinogenic therapeutically active polypeptide, has been chosen or determined for a given ailment, a skilled person would be able to achieve its expression, isolation and/or delivery using the reagents of the invention.

The invention also contemplates treatment of diseases in other animals including dogs, horses, cats and birds. Diseases in dogs include but are not limited to canine distemper (paramyxovirus), canine hepatitis (adenovirus Cav-1), kennel cough or laryngotracheitis (adenovirus Cav-2), infectious canine enteritis (coronavirus) and haemorrhagic enteritis (parvovirus).

Diseases in cats include but are not limited to viral rhinotracheitis (herpesvirus), feline caliciviral disease (calicivirus), feline infectious peritonitis (parvovirus) and feline leukaemia (feline leukaemia virus). Other viral diseases in horses and birds are also contemplated as being treatable using the methods and compositions of the invention. To this purpose, the use of microorganisms expressing recombinant interferons will be particularly preferred.

In a further aspect, the invention thus also provides a pharmaceutical composition comprising the host cell (strain, microbe) as taught herein, optionally transformed with the nucleic acid and/or the vector for a (preferably heterologous) expression product.

Preferably, such formulation comprise a therapeutically effective amount of the host cells of the invention and a pharmaceutically acceptable carrier, *i.e.,* one or more pharmaceutically acceptable carrier substances and/or additives, e.g., buffers, carriers, excipients, stabilisers, etc.

The term "pharmaceutically acceptable" as used herein is consistent with the art and means compatible with the other ingredients of a pharmaceutical composition and not deleterious to the recipient thereof.

The recombinant host cells of the invention can be suspended in a pharmaceutical formulation for administration to the human or animal having the disease to be treated. Such pharmaceutical formulations include but are not limited to live host cells and a medium suitable for administration. The recombinant host cells may be lyophilized in the presence of common excipients such as lactose, other sugars, alkaline and/or alkali earth stearate, carbonate and/or sulphate (for example, magnesium stearate, sodium carbonate and sodium sulphate), kaolin, silica, flavorants and aromas.

Host cells so-lyophilized may be prepared in the form of capsules, tablets, granulates and powders, each of which may be administered by the oral route.

Alternatively, some recombinant bacteria may be prepared as aqueous suspensions in suitable media, or lyophilized bacteria may be suspended in a suitable medium just prior to use, such medium including the excipients referred to herein and other excipients such as glucose, glycine and sodium saccharinate.

For oral administration, gastroresistant oral dosage forms may be formulated, which dosage forms may also include compounds providing controlled release of the host cells and thereby provide controlled release of the desired protein encoded therein. For example, the oral dosage form (including tablets, pellets, granulates, powders) may be coated with a thin layer of excipient (usually polymers, cellulosic derivatives and/or lipophilic materials) that resists dissolution or disruption in the stomach, but not in the intestine, thereby allowing transit through the stomach in favour of disintegration, dissolution and absorption in the intestine.

The oral dosage form may be designed to allow slow release of the host cells and of the recombinant protein thereof, for instance as controlled release, sustained release, prolonged release, sustained action tablets or capsules. These dosage forms usually contain conventional and well known excipients, such as lipophilic, polymeric, cellulosic, insoluble, swellable excipients. Controlled release formulations may also be used for any other delivery sites including intestinal, colon, bioadhesion or sublingual delivery (i.e., dental mucosal delivery) and bronchial delivery. When the compositions of the invention are to be administered rectally or vaginally, pharmaceutical formulations may include suppositories and creams. In this instance, the host cells are suspended in a mixture of common excipients also including lipids. Each of the aforementioned formulations are well known in the art and are described, for example, in the following references: Hansel et al. (1990, Pharmaceutical dosage forms and drug delivery systems, 5th edition, William and Wilkins); Chien 1992, Novel drug delivery system, 2nd edition, M. Dekker); Prescott et al. (1989, Novel drug delivery, J. Wiley & Sons); Cazzaniga et al., (1994, Oral delayed release system for colonic specific delivery, Int. J. Pharm.i08:7').

Preferably, an enema formulation may be used for rectal administration. The term "enema" is used to cover liquid preparations intended for rectal use. The enema may be usually supplied in single-dose containers and contains one or more active substances dissolved or dispersed in water, glycerol or macrogols or other suitable solvents.

Thus, according the invention, in a preferred embodiment, reduced-colonizing or non-colonizing strains of the bacterium, such as recombinant host cells encoding a desired gene may be administered to the animal or human via mucosal, e.g., an oral, nasal, rectal, vaginal or bronchial route by any one of the state-of-the art formulations applicable to the specific route. Dosages of host cells for administration will vary depending upon any number of factors including the type of bacteria and the gene encoded thereby, the type and severity of the disease to be treated and the route of administration to be used.

Thus, precise dosages cannot be defined for each and every embodiment of the invention, but will be readily apparent to those skilled in the art once armed with the present invention. The dosage could be anyhow determined on a case by case way by measuring the serum level concentrations of the recombinant protein after administration of predetermined numbers of cells, using well known methods, such as those known as ELISA or Biacore (See examples). The analysis of the kinetic profile and half life of the delivered recombinant protein provides sufficient information to allow the determination of an effective dosage range for the transformed host cells.

In an embodiment, when the host cells (strain, bacterium) express an antigen, the invention may thus also provide a vaccine. Preferably, the antigen may be capable of eliciting an immune response in and used as a vaccine in a human or animal.

The term "vaccine" identifies a pharmaceutically acceptable composition that, when administered in an effective amount to an animal or human subject, is capable of inducing antibodies to an immunogen comprised in the vaccine and/or elicits protective immunity in the subject.

The vaccine of the invention would comprise the host cells (strain, microbe) as taught herein, optionally transformed with the nucleic acids or vectors encoding the antigen and further optionally an excipient. Such vaccines may also comprise an adjuvant, i.e., a compound or composition that enhances the immune response to an antigen. Adjuvants include, but are not limited to, complete Freund's adjuvant, incomplete Freund's adjuvant, saponin, mineral gels such as aluminum hydroxide, surface active substances such as lysolecithin, pluronic polyols, polyanions, peptides, oil or hydrocarbon emulsions, and potentially useful pharmaceutically acceptable human adjuvants such as BCG (bacille Calmetle-Guerin) and *Corynebacterium parvum.*

In an embodiment, when the host cells (strain, bacterium) express a non-vaccinogenic prophylactically and/or therapeutically active peptide, polypeptide or protein, the invention may thus also provide a tool to deliver the same to a subject. The host cells may be administered as a suitable pharmaceutical formulation or composition. Hence, the formulation or composition would typically comprise the host cells (strain, microbe) as taught herein, optionally transformed with the nucleic acids or vectors encoding the non-vaccinogenic prophylactically and/or therapeutically active peptide, polypeptide or protein and further optionally a pharmaceutically acceptable excipient.

The invention is further illustrated with examples that are not to be considered limiting.

### Examples

### Example 1

### Construction of thyA deficient mutant strains

From *Streptococcus mutans, Lactobacillus acidophilus, Lactobacillus plantarum and Lactobacillus salivarius WCFS1,* we have cloned out 1000 bp regions flanking the thyA gene. The DNA sequence information for the genetic engineering of any *Streptococcus* and *Lactobacillus* strain in a way as described below are available from public sources (Table 1).

The strategy to generate thyA deficient strains employs double homologous recombination in the areas 1000 bp at the 5' end (SEQ ID N 1-4) and 1000 bp at the 3' end (SEQ ID N 5-8) of thyA, the "thyA target regions".

We have cloned these flanking DNA fragments in a derivative of pORI19 (Law et al., 1995 (J Bacteriol 177(24): 7011-8) a conditionally replication defective plasmid. Transformation of the plasmids in any of the *Streptococcus* and *Lactobacillus* strain only transfers the erythromycin resistance when a first homologous recombination occurs at either the 5' 1000 bp or at the 3' 1000 bp of the thyA target. A second homologous recombination at the 3' 1000 bp or at the 5' 1000 bp of the thyA target removes the thyA gene and yield the desired strain.

As an option, in between both 5' and 3' target regions additional genes are inserted, which will replace thyA in the thyA deficient strain.

### Evaluation of colonization

Wild type Streptococcus mutans, Lactobacillus acidophilus, Lactobacillus plantarum and Lactobacillus salivarius bacteria were transformed with a plasmids encoding a chloramphenicol (Cm) selection marker. Their respective thyA-deficient mutants were transformed with a plasmid encoding an erythromycin (Em) selection marker (the above: or vice versa).

Suspensions of the Cm+ wild type bacteria and of the Em+ thyA mutant bacteria were mixed and inoculated in the oral cavity, in the stomach through oral gavage or in the vagina. Daily samples from the oral cavity, stool and vagina were plated on either Cm or Em selective agar plates.

The relative presence of the Cm+ wild type bacteria and of the Em+ thyA mutant bacteria were determined by plate count and have shown a significant reduction of colonization of the Em+ thyA mutant bacteria in comparison to the Cm+ wild type bacteria.

**Table 1: Public sources for sequence information required for the construction of thyA deficient mutants of the indicated organisms.**

| Organism | Genome sequence | thyA gene | 5'thyA target | 3'thyA target |
|---|---|---|---|---|
| Streptococcus mutans UA 159 [1] | GenBank AE014133 Refseq NC 004350 | GeneID 1028296 | SEQ ID N 1 | SEQ ID N 5 |
| Lactobacillus acidophilus NCFM [2] | GenBank CP000033 Refseq NC 006841 | GeneID 3251830 | SEQ ID N 2 | SEQ ID N 6 |
| Lactobacillus plantarum WCFS1 [3] | GenBank AL935263 Refseq NC 004567 | GeneID 1064048 | SEQ ID N 3 | SEQ ID N 7 |
| Lactobacillus salivarius UCC118 [4] | GenBank CP000233 Refseq NC 007929 | GeneID 3976937 | SEQ ID N 4 | SEQ ID N 8 |

| | | | | |
|---|---|---|---|---|
| [1] (Ajdic et al., 2002 (PNAS 99(22): 14434-9) [2] (Altermann et al., 2005 (PNAS 102(11): 3906-12) [3] (Kleerebezem et al., 2003 (PNAS 100(4): 1990-5) [4] (Claesson et al., 2006 (PNAS 103(17): 6718-23) | | | | |

### Example 2: Effect of thyA deficiency on the colonizing capacity of Streptococcus mutans

*Streptococcus mutans* is a Gram positive, facultative anaerobic, lactic acid producing bacterium. It is a species that colonizes the human as well as hamster dental surface. As such it is an interesting candidate to serve as a host organism for delivery of therapeutic proteins to the oral mucosa. Hence, the effect of thyA deficiency on the colonizing capacity of *S. mutans* was investigated herein. We have used wild type *S. mutans* strain Clarke 1924 AL (LMG 14558; ATCC 25175; NCTC 10449) for all our experimentation.

### Example 2A: Construction of thyA- uidA+ Streptococcus mutans

Through double homologous recombination we have replaced thyA of *S. mutans* strain Clarke 1924 AL, with the uidA gene, encoding *E. coli* β-glucuronidase (GUS) (**Figure 1****,** **Figure 2**). The construction strategy uses a conditionally-replicative plasmid, pAGX0725, which was introduced in *Streptococcus mutans* Clarke 1924 AL under non-permissive circumstances. The plasmid pAGX0725 carries the uidA gene in-between 1kb regions cloned from chromosomal DNA upstream (thyA 5') or downstream (thyA 3') of thyA. Plating on erythromycin containing solid agar plates only allows for growth of those colonies in which a homologous recombination at either the thyA 5' or thyA 3' region had occurred. In this example, the recombination had taken place at both regions within the generations that led to growth of the first colonies, as was verified by PCR. The resulting *S. mutans* strain was named sAGX0108. The structure of the genetically modified region was verified by PCR and shows the presence of recombination events at thyA 5' and thyA 3', presence of the uidA gene as well as absence of thyA, all of which are in contrast with the structure of this region in *S. mutans* Clarke 1924 AL (**Figure 2**).

Genetically *S. mutans* sAGX0108 is thyA- uidA+. This phenotypically leads to strict growth dependence of *S. mutans* sAGX0108 for the addition of thymidine to the growth medium (**Figure 3**).

Further, *S. mutans* sAGX0108 shows β-glucuronidase activity, as can be observed by plating on agar plates (BHI+T) containing 5-bromo-4-chloro-3-indolyl-beta-D-glucuronic acid, cyclohexylammonium salt (X-Gluc). Wild type *S. mutans* Clarke 1924 AL remain white and S. *mutans* sAGX0108 colonies show a clear, intense blue precipitate.

### Example 2B: In vivo evaluation of the colonizing capacity of thyA+ and thyA- S. mutans

We investigated the colonizing capacity of thyA+ and thyA- *S. mutans* in hamsters. This was done by inoculating thyA+ and thyA- *S. mutans* in the cheek pouch of hamsters. For these *in vivo* colonization studies, a method to discriminate inoculated thyA+ and thyA- *S. mutans* from resident microflora in the hamster oral cavity is required. Because mixtures of thyA+ and thyA- *S. mutans* are inoculated, this method must also allow mutual discrimination of both strains.

We transformed wild type *S. mutans* Clarke 1924 AL with the plasmid pILPOL which carries an erythromycin resistance marker. *S. mutans* Clarke 1924 AL pILPOL can therefore be grown on erythromycin containing BHI solid agar plates. *S. mutans* sAGX0108 can be discriminated from the background on the basis of β-glucuronidase activity, which leads to blue staining following plating on 5-bromo-4-chloro-3-indolyl-beta-D-glucuronic acid (X-gluc) and thymidine (T) containing BHI solid agar plates. X-gluc is a substrate for β-glucuronidase which cleaves X-Gluc to produce colorless glucuronic acid and an intense blue precipitate of chloro-bromoindigo. Wild type *S*. *mutans* Clarke 1924 AL do not grow on erythromycin containing BHI solid agar plates and colonies remain white when plated on BHI solid agar plates containing X-gluc and thymidine.

Hence, when plating the respective strains, the following discriminatory properties of *S. mutans* strains Clarke 1924 AL pILPOL and sAGX0108 have been observed: *S. mutans* Clarke 1924 AL pILPOL shows normal growth on BHI solid agar plates containing erythromycin; *S. mutans* sAGX0108 shows intense blue staining of colonies when plated on BHI solid agar plates containing X-gluc and thymidine; *S. mutans* Clarke 1924 AL shows no growth on BHI solid agar plates containing erythromycin; and *S. mutans* Clarke 1924 AL shows no blue staining of colonies when plated on BHI solid agar plates containing X-gluc and thymidine.

Using the same approach, *S. mutans* Clarke 1924 AL pILPOL and *S. mutans* sAGX0108 can be mutually discriminated. While *S. mutans* sAGX0108 shows intense blue colonies, S. *mutans* Clarke 1924 AL pILPOL colonies remain white when plated on X-gluc and thymidine containing BHI solid agar plates. In contrast to *S. mutans* Clarke 1924 AL pILPOL, *S. mutans* sAGX0108 shows no growth when plated on BHI solid agar plates containing erythromycin. 50:50 mixtures of both strains plated on X-gluc and thymidine containing BHI solid agar plates therefore show equal numbers of blue and white colonies (representing *S. mutans* sAGX0108 and *S. mutans* Clarke 1924 AL pILPOL respectively) while plating of an identical amount of this mixture on BHI solid agar plates containing erythromycin renders a colony number which is half of the above number of blue and white colonies (representing *S. mutans* Clarke 1924 AL pILPOL only). Both *S. mutans* Clarke 1924 AL pILPOL and *S. mutans* sAGX0108 can in this way clearly be discriminated from background microflora.

This is demonstrated in the following test. *S. mutans* Clarke 1924 AL pILPOL and *S. mutans* sAGX0108 were grown overnight to saturation and cultures were diluted 10⁶ fold. From this diluted suspension, single strains and 50:50 mixtures of the two strains were plated on either BHI+E or BHI+T+X-Gluc solid agar plates, as indicated. Single strains were plated as 100µl volumes while for mixtures, 100µl of the 50:50 mixture was plated. Colony numbers are shown in the following Table 2.

**Table 2. Mutual discrimination of S. mutans Clarke 1924 AL pILPOL and S. mutans sAGX0108 (1: S. mutans Clarke 1924 AL pILPOL; 2: S. mutans sAGX0108; 1+2: S. mutans Clarke 1924 AL pILPOL + S. mutans sAGX0108).**

| | 1 | 2 | 1+2 |
|---|---|---|---|
| BHI + E | 30 colonies | 0 colonies | 44 colonies |
| BHI+T+C-Gluc | white: 79 colonies | blue: 134 colonies | white: 55 colonies |
| | | | blue: 44 colonies |

*S. mutans* Clarke 1924 AL pILPOL shows clear growth on BHI+E solid agar plates but does not show blue staining on BHI+T+X-Gluc solid agar plates. *S. mutans* sAGX0108 shows no growth on BHI+E solid agar plates but shows clear blue staining on BHI+T+X-Gluc solid agar plates. This enables the clear discrimination of a mixture of both strains.

We recorded the fate over time of thyA- *S. mutans* sAGX0108 and thyA+ *S. mutans* Clarke 1924 AL pILPOL following inoculation of a mixture of both strains in the cheek pouch of hamsters **(****Figure 4****).** Despite the fact that the inoculum contained over 4x more of the thyA-strain, samples from the dental surface as well as cheek pouch showed reduced colonizing capacity of the thyA- strain. For both strains, in both cheek pouch as well as on dental surface, an initial increase in concentration was observed between day 0 and day 1. Subsequently, concentration of both strains decreased (**Figure 4****,** Panels A and C) but this reduction was substantially faster for thyA- *S. mutans* sAGX0108 (**Figure 4****,** Panels B and D). Moreover, no thyA- *S. mutans* sAGX0108 could be detected in the cheek pouch on day 7 and 10 while thyA+ *S. mutans* Clarke 1924 AL pILPOL were still present at those time points.

The findings of the above experiment were confirmed in a separate experiment (example 2C).

### Example 2C: In vivo evaluation of the colonizing capacity of thyA+ and thyA- S. mutans

S. *mutans* Clarke 1924 AL pILPOL and *S. mutans* sAGX0108 were transformed with a plasmid conveying chloramphenicol resistance. This yielded strains *S. mutans* Clarke 1924 AL pILPOL Cm+ and *S. mutans* sAGX0108 Cm+. We however found that chlorampheincol resistance was unreliably inherited over generations when strains were grown at 33°C or higher, which is not compatible with experimentation in vivo in hamsters.

For this reason we used the same approach as described in Example 2B to follow the fate of both strains. Again, *S. mutans* Clarke 1924 AL pILPOL Cm+ and *S. mutans* sAGX0108 Cm+ could be mutually discriminated and both *S. mutans* Clarke 1924 AL pILPOL Cm+ and *S. mutans* sAGX0108 Cm+ can in this way clearly be discriminated from background microflora. We recorded the fate over time of thyA- *S. mutans* sAGX0108 Cm+ and thyA+ *S. mutans* Clarke 1924 AL pILPOL Cm+ following inoculation of a mixture of both strains in the cheek pouch of hamsters (**Figure 5**). Despite the fact that the inoculum contained over 2x more of the thyA- strain, samples from the dental surface as well as cheek pouch showed reduced colonizing capacity of the thyA- strain.

In the cheek pouch, both strains were stably maintained over 1 day. As from day 3 thyA- *S. mutans* sAGX0108 Cm+ could no longer be detected while thyA+ *S. mutans* Clarke 1924 AL pILPOL Cm+ were still present at all time points (**Figure 5**, Panels A and B)

On dental surface, the presence of both strains showed an initial decrease. As from day 3 no thyA- *S. mutans* sAGX0108 Cm+ could be detected while thyA+ *S. mutans* Clarke 1924 AL pILPOL Cm+ were still present at d3 and d7 (**Figure 5**, Panels C and D).

Relative to thyA+ *S. mutans* Clarke 1924 AL pILPOL Cm+, the presence of thyA- *S. mutans* sAGX0108 Cm+ showed an equally steep decrease in both cheek pouch as well as on dental surface (**Figure 5****,** Panels B and D).

The following Table 3 provides a summary of the strains used in the foregoing examples and their relevant characteristics (GUS: β-glucuronidase; Em: erythromycin; Cm chloramphenicol).

**Table 3**

| Strain | | Source | relevant characteristics | |
|---|---|---|---|---|
| | | GUS | Resistance to Em | Resistance to Cm |
| *S. mutans* strain Clarke 1924 AL | LMG | - | - | - |
| *S. mutans* strain Clarke 1924 AL pILPOL | AGX | - | + | - |
| *S. mutans* sAGX0108 | AGX | + | - | - |

The following Table 4 provides a summary of the abbreviations used here above.

| | |
|---|---|
| BHI | brain heart infusion broth |
| TF-BHI | thymidine free brain heart infusion broth |
| E | erythromycin |
| T | thymidine |
| X-Gluc | 5-bromo-4-chloro-3-indolyl-beta-D-glucuronic acid, cyclohexylammonium salt |
| GUS | β-glucuronidase |
| *thyA* | thymidylate synthase gene |
| uidA | E. coli β-glucuronidase gene |
| LMG | Laboratory of Microbiology Ghent |
| AGX | ActoGeniX |
| BAM9T | Carbonate and M9 salts buffer supplemented with amino acids and thymidine |
| BM9 | Carbonate and M9 salts buffer |

### References

Ajdic, D., W. M. McShan, et al. (2002). "Genome sequence of Streptococcus mutans UA159, a cariogenic dental pathogen." Proceedings of the National Academy of Sciences of the United States of America 99(22): 14434-9.
Allaker, R. P. and C. W. Douglas (2009). "Novel anti-microbial therapies for dental plaque-related diseases." International journal of antimicrobial agents 33(1): 8-13.
Altermann, E., W. M. Russell, et al. (2005). "Complete genome sequence of the probiotic lactic acid bacterium Lactobacillus acidophilus NCFM." Proceedings of the National Academy of Sciences of the United States of America 102(11): 3906-12.
Ausubel et al., Current Protocols in Molecular Biology, ed. Ausubel et al., Greene Publishing and Wiley-Interscience, New York, 1992 (with periodic updates)
Beighton, D. and H. Hayday (1982). "The streptococcal flora of the tongue of the monkey Macaca fascicularis." Archives of oral biology 27(4): 331-5.
Besier, S., A. Ludwig, et al. (2007). "Molecular analysis of the thymidine-auxotrophic small colony variant phenotype of Staphylococcus aureus." Int J Med Microbiol 297(4): 217-25.
Bigas, A., M. E. Garrido, et al. (2006). "Colonization capacity and serum bactericidal activity of Haemophilus parasuis thy mutants." Int Microbiol 9(4): 297-301.
Cazzaniga et al., Oral delayed release system for colonic specific delivery, Int. J. Pharm. 1994.
Chien, Novel drug delivery system, 2nd edition, M. Dekker, 1992
Claesson, M. J., Y. Li, et al. (2006). "Multireplicon genome architecture of Lactobacillus salivarius." Proceedings of the National Academy of Sciences of the United States of America 103(17): 6718-23.
Coykendall, A. L., D. Bratthall, et al. (1976). "Serological and genetic examination of some nontypical Streptococcus mutans strains." Infection and immunity 14(3): 667-70.
Davis, B. D. et al., Microbiology, 3rd edition, Harper & Row, publishers, Philadelphia, Pa., 1980.
Dent, V. E., J. M. Hardie, et al. (1978). "Streptococci isolated from dental plaque of animals." The Journal of applied bacteriology 44(2): 249-58.
Finegold, S. M., D. J. Flora, et al. (1975). "Fecal bacteriology of colonic polyp patients and control patients." Cancer research 35(11 Pt. 2): 3407-17.
Gehring, F., E. J. Karle, et al. (1976). "[The occurrence of Streptococcus mutans variants in man and laboratory animals]." Deutsche zahnarztliche Zeitschrift 31(1): 18-21.
Hamada, S., N. Masuda, et al. (1980). "Isolation and serotyping of Streptococcus mutans from teeth and feces of children." Journal of clinical microbiology 11(4): 314-8.
Hamada, S. and H. D. Slade (1980). "Biology, immunology, and cariogenicity of Streptococcus mutans." Microbiological reviews 44(2): 331-84.
Hamady, Z. Z., N. Scott, et al. (2009). "Xylan regulated delivery of human keratinocyte growth factor-2 to the inflamed colon by the human anaerobic commensal bacterium Bacteroides ovatus." Gut (ePub ahead of print).
Hansel et al., Pharmaceutical dosage forms and drug delivery systems, 5th edition, William and Wilkins, 1990.
Hasselbring, B. M., C. A. Page, et al. (2006). "Transposon mutagenesis identifies genes associated with Mycoplasma pneumoniae gliding motility." J Bacteriol 188(17): 6335-45.
Huber, G., J. Van Houte, et al. (1977). "Relationship between the populations of Streptococcus mutans in the mouth and feces of conventional Sprague-Dawley rats." Journal of dental research 56(12): 1614-9.
Innis et al., PCR Protocols: A Guide to Methods and Applications, Academic Press: San Diego, 1990.
Kilian, M., E. Theilade, et al. (1971). "Isolation of Streptococcus mutans from human faeces." Archives of oral biology 16(5): 553-4.
Kleerebezem, M., J. Boekhorst, et al. (2003). "Complete genome sequence of Lactobacillus plantarum WCFS1." Proceedings of the National Academy of Sciences of the United States of America 100(4): 1990-5.
Law, J., G. Buist, et al. (1995). "A system to generate chromosomal mutations in Lactococcus lactis which allows fast analysis of targeted genes." J Bacteriol 177(24): 7011-8.
Lehner, T., S. J. Challacombe, et al. (1975). "Immunological and bacteriological basis for vaccination against dental caries in rhesus monkeys." Nature 254(5500): 517-20.
Liljemark, W. F., S. V. Schauer, et al. (1978). "Compounds which affect the adherence of Streptococcus sanguis and Streptococcus mutans to hydroxyapatite." Journal of dental research 57(2): 373-9.
Maruyama, F., M. Kobata, et al. (2009). "Comparative genomic analyses of Streptococcus mutans provide insights into chromosomal shuffling and species-specific content." BMC genomics 10: 358.
Perez-Martinez, G., J. Kok, et al. (1992). "Protein export elements from Lactococcus lactis." Mol Gen Genet 234(3): 401-11.
Prescott et al., Novel drug delivery, J. Wiley & Sons, 1998
Proctor, R. A., P. van Langevelde, et al. (1995). "Persistent and relapsing infections associated with small-colony variants of Staphylococcus aureus." Clin Infect Dis 20(1): 95-102.
Sambrook et al., Molecular Cloning: A Laboratory Manual, 2nd ed., vol. 1-3, ed. Cold Spring Harbor Laboratory Press, Cold Spring Harbor, N.Y., 1989
Samuel, J. L. (1982). "The normal flora of the mouths of macropods (Marsupialia: macropodidae)." Archives of oral biology 27(2): 141-6.
Sasaki, Y., Y. Ito, et al. (2004). "ThyA as a selection marker in construction of food-grade host-vector and integration systems for Streptococcus thermophilus." Appl Environ Microbiol 70(3): 1858-64.
Sibakov, M., T. Koivula, et al. (1991). "Secretion of TEM beta-lactamase with signal sequences isolated from the chromosome of Lactococcus lactis subsp. lactis." Appl Environ Microbiol 57(2): 341-8.
Spanu, T., L. Romano, et al. (2005). "Recurrent ventriculoperitoneal shunt infection caused by small-colony variants of Staphylococcus aureus." Clin Infect Dis 41(5): e48-52.
Steidler, L., W. Hans, et al. (2000). "Treatment of murine colitis by Lactococcus lactis secreting interleukin-10." Science 289: 1352-5.
Steidler, L., S. Neirynck, et al. (2003). "Biological containment of genetically modified Lactococcus lactis for intestinal delivery of human interleukin 10." Nat. Biotechnol. 21: 785-9.
Steidler, L., K. Robinson, et al. (1998). "Mucosal delivery of murine interleukin-2 (IL-2) and IL-6 by recombinant strains of Lactococcus lactis coexpressing antigen and cytokine." Infect Immun 66(7): 3183-9.
Thomson, L. A., W. H. Bowen, et al. (1979). "Simultaneous implantation of five serotypes of Streptococcus mutans in gnotobiotic rats." Caries research 13(1): 9-17.
Unsworth, P. F. (1980). "The isolation of streptococci from human faeces." The Journal of hygiene 85(1): 153-64.
Wunder, J. A., W. W. Briner, et al. (1976). "Identification of the cultivable bacteria in dental plaque from the beagle dog." Journal of dental research 55(6): 1097-102.

## Claims

1. A non-pathogenic, non-invasive Gram-positive bacterium comprising:
(a) a defective thymidylate synthase (*thyA*) gene;
(b) a recombinant nucleic acid comprising a promoter operably linked to one or more open reading frames (ORFs) encoding one or more heterologous expression products which are capable of eliciting a prophylactic and/or therapeutic response in a human or animal,
wherein said bacterium has a reduced capacity of colonizing a mucosa of said human or animal, in comparison to its wild type ancestor, and
wherein said promoter in the recombinant nucleic acid is the native promoter of a *Lactococcus* gene selected from the group consisting of: glyceraldehyde-3-phosphate dehydrogenase (*gapB*), non-heme iron-binding ferritin (*dpsA*), pyruvate kinase (*pyk*), phosphopyruvate hydratase (eno), dipeptidase PepV (*pepV*), superoxide dismutase (*sodA*), ribosomal protein L19 (*rplS*), HU-like DNA binding protein (*hllA*), and 50S ribosomal protein L28 (*rpmB*).

2. The bacterium according to claim 1, wherein the said recombinant nucleic acid encoding the one or more heterologous expression products is comprised in a vector.

3. The bacterium according to claim 1 or claim 2, wherein the said one or more heterologous expression products is a peptide, polypeptide or protein, preferably an antigen and/or non-vaccinogenic prophylactically and/or therapeutically active peptide, polypeptide or protein.

4. The bacterium according to claim 3, wherein the said antigen is capable of eliciting an immune tolerance response in the said human or said animal.

5. The bacterium according to any one of claims 1 to 4, wherein the said one or more heterologous expression products is selected from: insulin, a growth hormone, prolactin, calcitonin, luteinising hormone, parathyroid hormone, somatostatin, thyroid stimulating hormone, vasoactive intestinal polypeptide, a cytokine, a receptor or antagonist thereof, hIL-10, GLP-2, GLP-1, TFF or hPYY.

6. A method for reducing or abolishing the colonization capacity of a bacterium, wherein said bacterium is capable of residing in the mucosa of a human or animal and wherein said bacterium comprises a recombinant nucleic acid which encodes one or more heterologous expression products capable of eliciting a prophylactic and/or therapeutic response in said human or animal, wherein said recombinant nucleic acid comprises a promoter which is a native promoter of a *Lactococcus* gene selected from the group consisting of: glyceraldehyde-3-phosphate dehydrogenase (*gapB*), non-heme iron-binding ferritin (*dpsA*), pyruvate kinase (*pyk*), phosphopyruvate hydratase (*eno*), dipeptidase PepV (*pepV*), superoxide dismutase (*sodA*), ribosomal protein L19 (*rplS*), HU-like DNA binding protein (*hllA*), and 50S ribosomal protein L28 (*rpmB*)*,* wherein said method comprises rendering defective a thymidylate synthase (*thyA*) gene in said bacterium, and wherein said one or more heterologous expression products is selected from: insulin, a growth hormone, prolactin, calcitonin, luteinising hormone, parathyroid hormone, somatostatin, thyroid stimulating hormone, vasoactive intestinal polypeptide, a cytokine, a receptor or antagonist thereof, hIL-10, GLP-2, GLP-1, TFF or hPYY.

7. The bacterium according to any one of claims 1 to 5 or the method according to claim 6, wherein said defective *thyA* gene is an endogenous *thyA* gene, preferably wherein the endogenous *thyA* gene is chromosomally located.

8. The bacterium or method according to any one of claims 1 to 7, wherein the defective *thyA* gene is a recombinant defective *thyA* gene, preferably wherein the *thyA* gene is situated in the chromosome and is inactivated by gene disruption.

9. The bacterium or method according to any one of claims 1 to 8, wherein the bacterium is thymidylate synthase deficient.

10. The bacterium or method according to any one of claims 1 to 9, wherein said bacterium elicits a prophylactic and/or therapeutic effect in said human or said animal.

11. The method according to any one of claims 6 to 10, wherein the bacterium further comprises suitable regulatory sequences operably linked to one or more ORFs encoding the said one or more heterologous expression products.

12. The bacterium or method according to anyone of claims 1 to 10, wherein the bacterium further comprises a sequence encoding a secretion signal in phase with a polypeptide encoded by the said one or more ORFs.

13. The bacterium or method according to anyone of claims 5 to 12, wherein the cytokine is selected from: an interleukin, GM-CSF, M-CSF, SCF, IFNs, EPO, G-CSF, LIF, OSM, CNTF, GH, PRL, a TNF family member, TNFα, TNFβ, CD40, CD27, a FAS ligand, an IL-1 family member, a fibroblast growth factor family member, a platelet-derived growth factor, a transforming growth factor, a nerve growth factor, and an epidermal growth factor family member.

14. The bacterium or method according to claim 13, wherein the interleukin is selected from:
IL-2, IL-3, IL-4, IL-5, IL-6, IL-7, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34 and IL-35.

15. The method according to any of the claims 6 to 14, wherein the said bacterium is a non-pathogenic, non-invasive Gram-positive bacterium.

16. The method according to claim 15, wherein the said bacterium is selected from the group consisting of *Bacteroides* sp., *Clostridium* sp., *Fusobacterium* sp., *Eubacterium* sp., *Ruminococcus* sp., *Peptococcus* sp., *Peptostreptococcus* sp., *Streptococcus* sp., *Bifidobacterium* sp., *Escherichia* sp., *Lactobacillus* sp., and any subspecies or strains thereof.

17. The bacterium according to any one of claims 1 to 5, 7 to 10 or 12 to 14, wherein the said bacterium is selected from the group consisting of *Streptococcus* sp., *Lactobacillus* sp., or any subspecies or strains thereof.

18. A pharmaceutical composition comprising the bacterium according to any one of claims 1 to 5, 7 to 10 or 12 to 14 and a pharmaceutically acceptable carrier.

19. A starter culture, inoculum, lyophilized composition, frozen liquid composition, or food and feed composition comprising the bacterium according to any one of claims 1 to 5, 7 to 10 or 12 to 14.

20. The bacterium according to any one of claims 1 to 5, 7 to 10 or 12 to 14 for use in treating a disease selected from the group consisting of: an inflammatory bowel disease, an autoimmune disease, a neurological disorder, cancer, osteoporosis, diabetes, cardiovascular disease, atherosclerosis, hemophilia, degenerative liver disease, a pulmonary disease, obesity, and pathogen infections.

## Patentansprüche

1. Nicht-pathogenes, nicht-invasives grampositives Bakterium, umfassend:
(a) ein defektes Thymidylat-Synthase-Gen (*thyA-Gen*);
(b) rekombinante Nukleinsäure, die einen Promotor umfasst, der funktionell mit einem oder mehreren offenen Leserastern (ORF) verknüpft ist, die für ein oder mehrere heterologe Expressionsprodukte kodieren, die fähig sind, in einem Menschen oder Tier eine prophylaktische und/oder therapeutische Reaktion hervorzurufen,
wobei das Bakterium im Vergleich zu seinem Wildtyp-Vorfahren eine verringerte Fähigkeit zur Kolonisierung einer Schleimhaut des Menschen oder Tieres aufweist, und
wobei der Promotor in der rekombinanten Nukleinsäure der native Promotor eines *Lactococcus-*Gens ist, das aus der Gruppe bestehend aus Glyceraldehyd-3-phosphat-Dehydrogenase (*gapB*), Nicht-Häm-Eisen bindendem Ferritin (*dpsA*), Pyruvatkinase (*pyk*), Phosphopyruvathydratase (*eno*), Dipeptidase PepV (*pepV*)*,* Superoxiddismutase (*sodA*), ribosomalem Protein L19 (*rplS*), Hu-ähnlichem DNA bindendem Protein (*hllA*) und 50S-ribosomalem Protein L28 (*rpmB*) ausgewählt ist.

2. Bakterium nach Anspruch 1, wobei die rekombinante Nukleinsäure, die für das eine oder die mehreren heterologen Expressionsprodukte kodiert, in einem Vektor enthalten ist.

3. Bakterium nach Anspruch 1 oder Anspruch 2, wobei das eine oder die mehreren heterologen Expressionsprodukte ein Peptid, Polypeptid oder Protein, vorzugsweise ein Antigen und/oder ein nicht vakzinogenes prophylaktisch und/oder therapeutisch aktives Peptid, Polypeptid oder Protein, sind.

4. Bakterium nach Anspruch 3, wobei das Antigen fähig ist, in dem Menschen oder dem Tier eine Immuntoleranzantwort hervorzurufen.

5. Bakterium nach einem der Ansprüche 1 bis 4, wobei das eine oder die mehreren heterologen Expressionsprodukte aus Insulin, einem Wachstumshormon, Prolactin, Calcitonin, luteinisierendem Hormon, Parathyroidhormon, Somatostatin, Thyroid stimulierendem Hormon, vasoaktivem intestinalem Polypeptid, einem Cytokin, einem Rezeptor oder Antagonisten davon, hIL-10, GLP-2, GLP-1, TFF oder hPYY ausgewählt sind.

6. Verfahren zum Reduzieren oder Aufheben der Kolonisierungsfähigkeit eines Bakteriums, wobei das Bakterium fähig ist, sich in der Schleimhaut eines Menschen oder Tieres aufzuhalten, und wobei das Bakterium eine rekombinante Nukleinsäure umfasst, die für ein oder mehrere heterologe Expressionsprodukte kodiert, die fähig sind, in dem Menschen oder Tier eine prophylaktische und/oder therapeutische Reaktion hervorzurufen, wobei die rekombinante Nukleinsäure einen Promotor umfasst, der ein nativer Promotor eines *Lactococcus*-Gens ist, das aus der Gruppe bestehend aus Glyceraldehyd-3-phosphat-Dehydrogenase (*gapB*)*,* Nicht-Häm-Eisen bindendem Ferritin (*dpsA*)*,* Pyruvatkinase (*pyk*)*,* Phosphopyruvathydratase (*eno*)*,* Dipeptidase PepV (*pepV*)*,* Superoxiddismutase (*sodA*), ribosomalem Protein L19 (*rplS*), HU-ähnlichem DNA bindendem Protein (*hllA*) und 50S-ribosomalem Protein L28 (*rpmB*) ausgewählt ist, wobei das Verfahren das Erzeugen eines defekten Thymidylat-Synthase-Gens (*thyA-Gen*) in dem Bakterium umfasst, und wobei das eine oder die mehreren heterologen Expressionsprodukte aus Insulin, einem Wachstumshormon, Prolactin, Calcitonin, luteinisierendem Hormon, Parathyroidhormon, Somatostatin, thyroidstimulierendem Hormon, vasoaktivem intestinalem Polypeptid, einem Cytokin, einem Rezeptor oder Antagonisten davon, hIL-10, GLP-2, GLP-1, TFF oder hPYY ausgewählt sind.

7. Bakterium nach einem der Ansprüche 1 bis 5 oder Verfahren nach Anspruch 6, wobei das defekte *ThyA-Gen* ein endogenes *ThyA-Gen* ist, wobei das endogene *ThyA-Gen* vorzugsweise chromosomal lokalisiert ist.

8. Bakterium oder Verfahren nach einem der Ansprüche 1 bis 7, wobei das defekte *ThyA-*Gen ein rekombinantes defektes *ThyA-Gen* ist, wobei sich das *ThyA-Gen* vorzugsweise im Chromosom befindet und durch Genunterbrechung inaktiviert wird.

9. Bakterium oder Verfahren nach einem der Ansprüche 1 bis 8, wobei das Bakterium einen Thymidylat-Synthase-Mangel aufweist.

10. Bakterium oder Verfahren nach einem der Ansprüche 1 bis 9, wobei das Bakterium bei dem Menschen oder dem Tier eine prophylaktische und/oder therapeutische Wirkung hervorruft.

11. Verfahren nach einem der Ansprüche 6 bis 10, wobei das Bakterium ferner geeignete regulatorische Sequenzen umfasst, die funktionell mit einem oder mehreren ORF verknüpft sind, die für das eine oder die mehreren heterologen Expressionsprodukte kodieren.

12. Bakterium oder Verfahren nach einem der Ansprüche 1 bis 10, wobei das Bakterium ferner eine Sequenz umfasst, die phasengleich mit einem von dem einen oder den mehreren ORF kodierten Polypeptid für ein Sekretionssignal kodiert.

13. Bakterium oder Verfahren nach einem der Ansprüche 5 bis 12, wobei das Cytokin aus einem Interleukin, GM-CSF, M-CSF, SCF, IFN, EPO, G-CSF, LIF, OSM, CNTF, GH, PRL, einem Mitglied der TNF-Familie, TNFα, TNFβ, CD40, CD27, einem FAS-Liganden, einem Mitglied der IL-1-Familie, einem Mitglied der Familie der Fibroblasten-Wachstumsfaktoren, einem PDGF (Platelet-derived growth factor), einem transformierenden Wachstumsfaktor, einem Nervenwachstumsfaktor und einem Mitglied der Familie der epidermalen Wachstumsfaktoren ausgewählt ist.

14. Bakterium oder Verfahren nach Anspruch 13, wobei das Interleukin aus IL-2, IL-3, IL-4, IL-5, IL-6, IL-7, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33, IL-34 und IL-35 ausgewählt ist.

15. Verfahren nach einem der Ansprüche 6 bis 14, wobei das Bakterium ein nicht-pathogenes, nicht-invasives grampositives Bakterium ist.

16. Verfahren nach Anspruch 15, wobei das Bakterium aus der Gruppe bestehend aus *Bacteroides* sp., *Clostridium* sp., *Fusobacterium* sp., *Eubacterium* sp., *Ruminococcus* sp., *Peptococcus* sp., *Peptostreptococcus* sp., *Streptococcus* sp., *Bifidobacterium* sp., *Escherichia* sp., *Lactobacillus* sp. und beliebigen Subspezies oder Stämmen davon ausgewählt ist.

17. Bakterium nach einem der Ansprüche 1 bis 5, 7 bis 10 oder 12 bis 14, wobei das Bakterium aus der Gruppe bestehend aus *Streptococcus* sp., *Lactobacillus* sp. oder beliebigen Subspezies oder Stämmen davon ausgewählt ist.

18. Pharmazeutische Zusammensetzung, umfassend das Bakterium nach einem der Ansprüche 1 bis 5, 7 bis 10 oder 12 bis 14 und einen pharmazeutisch verträglichen Träger.

19. Starterkultur, Inokulum, lyophilisierte Zusammensetzung, gefrorene flüssige Zusammensetzung oder Nahrungsmittel- und Futterzusammensetzung, umfassend das Bakterium nach einem der Ansprüche 1 bis 5, 7 bis 10 oder 12 bis 14.

20. Bakterium nach einem der Ansprüche 1 bis 5, 7 bis 10 oder 12 bis 14 zur Verwendung bei der Behandlung einer Krankheit, die aus der Gruppe bestehend aus einer entzündlichen Darmerkrankung, einer Autoimmunerkrankung, einer neurologischen Störung, Krebs, Osteoporose, Diabetes, kardiovaskuläre Erkrankung, Atherosklerose, Hämophilie, degenerativer Lebererkrankung, einer Lungenerkrankung, Fettleibigkeit und pathogenen Infektionen ausgewählt ist.

## Revendications

1. Bactérie à Gram positif non pathogène, non invasive comprenant :
(a) un gène déficient de thymidylate synthase (*thyA*) ;
(b) un acide nucléique recombiné comprenant un promoteur lié de manière fonctionnelle à un ou plusieurs cadres de lecture ouverts (ORF) codant pour un ou plusieurs produits d'expression hétérologues qui sont capables de provoquer une réponse prophylactique et/ou thérapeutique chez un humain ou un animal,
dans laquelle ladite bactérie a une capacité réduite de colonisation d'une muqueuse dudit humain ou animal, par comparaison avec son ancêtre de type sauvage, et
dans laquelle ledit promoteur dans l'acide nucléique recombiné est le promoteur natif d'un gène de *Lactococcus* choisi dans le groupe constitué de : glycéraldéhyde-3-phosphate déshydrogénase (*gapB*)*,* ferritine de liaison de fer non hémique (*dpsA*)*,* pyruvate kinase (*pyk*)*,* phosphopyruvate hydratase (*eno*), dipeptidase PepV (*pepV*)*,* superoxyde dismutase (*sodA*), protéine ribosomique L19 (*rplS*), protéine de liaison d'ADN de type HU (*hllA*), et protéine ribosomique 50S L28 (*rpmB*).

2. Bactérie selon la revendication 1, dans laquelle ledit acide nucléique recombiné codant pour le ou les produits d'expression hétérologues est compris dans un vecteur.

3. Bactérie selon la revendication 1 ou la revendication 2, dans laquelle ledit ou lesdits produits d'expression hétérologues sont un peptide, un polypeptide ou une protéine, de préférence un antigène et/ou un(e) peptide, polypeptide ou protéine, non vaccinogène, actif(active) sur le plan prophylactique et/ou thérapeutique.

4. Bactérie selon la revendication 3, dans laquelle ledit antigène est capable de provoquer une réponse de tolérance immunitaire chez ledit humain ou ledit animal.

5. Bactérie selon l'une quelconque des revendications 1 à 4, dans laquelle ledit ou lesdits produits d'expression hétérologues sont choisis parmi : insuline, une hormone de croissance, prolactine, calcitonine, hormone lutéinisante, hormone de parathyroïde, somatostatine, hormone de stimulation thyroïdienne, polypeptide intestinal vasoactif, une cytokine, un récepteur ou antagoniste de ceux-ci, hIL-10, GLP-2, GLP-1, TFF ou hPYY.

6. Procédé de réduction ou d'abolition de la capacité de colonisation d'une bactérie, dans lequel ladite bactérie est capable de résider dans la muqueuse d'un humain ou animal et dans lequel ladite bactérie comprend un acide nucléique recombiné qui code pour un ou plusieurs produits d'expression hétérologues capables de provoquer une réponse prophylactique et/ou thérapeutique chez ledit humain ou animal, dans lequel ledit acide nucléique recombiné comprend un promoteur qui est un promoteur natif d'un gène de *Lactococcus* choisi dans le groupe constitué de : glycéraldéhyde-3-phosphate déshydrogénase (*gapB*)*,* ferritine de liaison de fer non hémique (*dpsA*)*,* pyruvate kinase (*pyk*)*,* phosphopyruvate hydratase (*eno*), dipeptidase PepV (*pepV*)*,* superoxyde dismutase (*sodA*), protéine ribosomique L19 (*rplS*), protéine de liaison d'ADN de type HU (*hllA*), et protéine ribosomique 50S L28 (*rpmB*), dans lequel ledit procédé comprend le fait de rendre déficient un gène de thymidylate synthase (*thyA*) dans ladite bactérie, et dans lequel ledit ou lesdits produits d'expression hétérologues sont choisis parmi : insuline, une hormone de croissance, prolactine, calcitonine, hormone lutéinisante, hormone de parathyroïde, somatostatine, hormone de stimulation thyroïdienne, polypeptide intestinal vasoactif, une cytokine, un récepteur ou antagoniste de celle-ci, hIL-10, GLP-2, GLP-1, TFF ou hPYY.

7. Bactérie selon l'une quelconque des revendications 1 à 5 ou procédé selon la revendication 6, dans lequel ledit gène déficient de *thyA* est un gène de *thyA* endogène, de préférence dans lequel le gène de *thyA* endogène est chromosomiquement situé.

8. Bactérie ou procédé selon l'une quelconque des revendications 1 à 7, dans lequel le gène déficient de *thyA* est un gène déficient recombiné de *thyA,* de préférence dans lequel le gène de *thyA* est situé dans le chromosome et est inactivé par disruption génique.

9. Bactérie ou procédé selon l'une quelconque des revendications 1 à 8, dans lequel la bactérie est déficiente en thymidylate synthase.

10. Bactérie ou procédé selon l'une quelconque des revendications 1 à 9, dans lequel ladite bactérie provoque un effet prophylactique et/ou thérapeutique chez ledit humain ou ledit animal.

11. Procédé selon l'une quelconque des revendications 6 à 10, dans lequel la bactérie comprend en outre des séquences régulatrices appropriées liées de manière fonctionnelle à un ou plusieurs ORF codant pour ledit ou lesdits produits d'expression hétérologues.

12. Bactérie ou procédé selon l'une quelconque des revendications 1 à 10, dans lequel la bactérie comprend en outre une séquence codant pour un signal de sécrétion en phase avec un polypeptide codé par ledit ou lesdits ORF.

13. Bactérie ou procédé selon l'une quelconque des revendications 5 à 12, dans lequel la cytokine est choisie parmi : une interleukine, GM-CSF, M-CSF, SCF, des IFN, EPO, G-CSF, LIF, OSM, CNTF, GH, PRL, un membre de la famille TNF, TNFα, TNFβ, CD40, CD27, un ligand FAS, un membre de la famille d'IL-1, un membre de la famille du facteur de croissance de fibroblaste, un facteur de croissance dérivé de plaquettes, un facteur de croissance transformant, un facteur de croissance nerveuse, et un membre de la famille du facteur de croissance épidermique.

14. Bactérie ou procédé selon la revendication 13, dans lequel l'interleukine est choisie parmi : IL-2, IL-3, IL-4, IL-5, IL-6, IL-7, IL-9, IL-10, IL-11, IL-12, IL-13, IL-14, IL-15, IL-16, IL-17,
IL-18, IL-19, IL-20, IL-21, IL-22, IL-23, IL-24, IL-25, IL-26, IL-27, IL-28, IL-29, IL-30, IL-31, IL-32, IL-33, IL-34 et IL-35.

15. Procédé selon l'une quelconque des revendications 6 à 14, dans lequel ladite bactérie est une bactérie à Gram positif non pathogène, non invasive.

16. Procédé selon la revendication 15, dans lequel ladite bactérie est choisie dans le groupe constitué de *Bacteroides* sp., *Clostridium* sp., *Fusobacterium* sp., *Eubacterium* sp., *Ruminococcus* sp., *Peptococcus* sp., *Peptostreptococcus* sp., *Streptococcus* sp., *Bifidobacterium* sp., *Escherichia* sp., *Lactobacillus* sp., et n'importe quelles sous-espèces ou souches de ceux-ci.

17. Bactérie selon l'une quelconque des revendications 1 à 5, 7 à 10 ou 12 à 14, dans laquelle ladite bactérie est choisie dans le groupe constitué de *Streptococcus* sp., *Lactobacillus* sp., ou n'importe quelles sous-espèces ou souches de ceux-ci.

18. Composition pharmaceutique comprenant la bactérie selon l'une quelconque des revendications 1 à 5, 7 à 10 ou 12 à 14 et un véhicule acceptable sur le plan pharmaceutique.

19. Culture de départ, inoculation, composition lyophilisée, composition liquide congelée, ou composition pour consommation humaine et animale comprenant la bactérie selon l'une quelconque des revendications 1 à 5, 7 à 10 ou 12 à 14.

20. Bactérie selon l'une quelconque des revendications 1 à 5, 7 à 10 ou 12 à 14 pour une utilisation dans le traitement d'une maladie choisie dans le groupe constitué de : une maladie intestinale inflammatoire, une maladie auto-immune, un trouble neurologique, cancer, ostéoporose, diabète, maladie cardiovasculaire, athérosclérose, hémophilie, maladie hépatique dégénérative, une maladie pulmonaire, obésité, et infections par un pathogène.


### SEQUENCE LISTING

<110> Intrexon Actobiotics N.V.
<120> REDUCED COLONIZATION OF MICROBES AT THE MUCOSA
<130> INX16605PCTEPD1
<140> Not yet assigned
   <141> 2009-09-29
<150> EP 09736880.7
   <151> 2009-09-29
<150> PCT/EP2009/062647
   <151> 2009-09-29
<150> EP 08105458.7
   <151> 2008-09-29
<160> 9
<170> PatentIn version 3.3
<210> 1
   <211> 1000
   <212> DNA
   <213> Streptococcus mutans UA 159
<400> 1
   caaaattcac cttaagtttt tcctgcaaat cagatgggac ttgcttatcc atgattttgt 60
   taaagccttt aagagctagt ttaggaaaag gaagatgaaa gcaaaaagct gcaaaatcag 120
   ttaaagaaac atcaaaacgt ttttgatatt ctgcccaagt cgttttcagc atatccaaat 180
   attgtttggt agaatacata ccattaacat aaggtgttgt tgtataattt ggccgccaaa 240
   aatccataat atcacgtgtc tgagctaagg tctcatcatg aagaataaga atacggggat 300
   cttttttgac cagcatggca atgctgccgg ctccttgagt agattctccg ggagttccaa 360
   ttccgtattt ggcaatatcg ctagcaagaa caagcacacg cgtgtcagga tgtttttcaa 420
   catgcagttt agcatagttg agagctgcag tagcactgta gcatgcctct ttcatctcaa 480
   agctgcgcgc aaaaggctga atacctaata aggaatgcac ataaacagct ccagccttac 540
   tttgatcaac gctggattcg gtagctaaaa tgaccatatc aattttttct ttatcctctg 600
   cagtcagaat ttcattggct gatccggctg ctagggtgac aacgtcgtcg gtaataggag 660
   cgatgctaag agcattcagc aaaagtcctt tactgaattt ttggggatct tcgccacggg 720
   cctcagctaa atctttcata ttcaggacat attggctgct tgtaaaacca attttatcaa 780
   tcccaattct cataagtcaa tccttctttt cttttttatc tgatttcttt tttgtaaaat 840
   gtcatagtta ttttatcaca atttaaacta aaggtaatga ataaactttc aaggattcaa 900
   tttttgggag aatctgtcag atcagacgaa acctattttt tctgctataa tatctaaaag 960
   aagtttaaat tattttctta gaatcataga aggagaagca 1000
<210> 2
   <211> 1000
   <212> DNA
   <213> Lactobacillus acidophilus NCFM
<400> 2
   atttttcttg gtgtaggcaa cggtggaaag ccaattggtt ttagcaattt atgggctcat      60
   ggcggtttct ttactggcgg agtaaaaggc tttttcttct cgatgtcaat tattgttggt     120
   tcatatgaag gaattgagtt gctaggcatt tcggcaggcg aggtggctaa cccgcaaaaa     180
   gcaattgtaa agagtgtcaa gtctgttctt ttgagaattt taattttcta cgttggtgca     240
   atttttgtga ttgtaactat ttatccgtgg aataagttaa gtagcttagg ttctcctttt     300
   gtaactacct ttgctaaggt aggaatcact gctgctgctt cgattattaa cttcgttgta     360
   ttaactgcag cattgtcagg agctaactcg gggatttatt catcaagtag aatgctattt     420
   aaattgtctc atgataatga agcacctagt gtatttaagc atatttctaa gagaattgtg     480
   cctgatcgcg ccattatggg gatttctggt gggattttta ttgggtttat tttaaatata     540
   attgcatctc aatttaatca ttcggcatca gatttatttg tgattgtctt cagttcatca     600
   gttttaccag gaatgattcc atggtttgta attctattgg ctgagttaag atttagaaga     660
   cataatcaag atatgatgaa agatcacccg ttcaaattgc cgttatatcc attttctaat     720
   tacttcgcat ttttaatgct gttagtaatt gttatcttta tgtttattaa tccagatact     780
   agaatttcag taattaccgg agcattggta ttaattgtgg ctacaattgt ttatttagtt     840
   agacataaag atgaatttag taaaaataat taatgattaa gaagtcttga aatttcaggg     900
   ctttttattt ttaccaaata ctaaatattg atacttgcat tatcaaaaat actagatcta     960
   tggtatctta aaaagaaatg atacttaaag ggtgagataa                          1000
<210> 3
   <211> 1000
   <212> DNA
   <213> Lactobacillus plantarum WCFS1
<400> 3
   ttcgctcggt cagcagcgtt tgggtaagaa agtgatcgag ttaaaggacg cgagcttgca      60
   gtttgatcgg caaaccatcc tggatcactt ttcgatgttg atccaagcca atgaccgcat     120
   cggtatcact ggaatcaacg gtgccgggaa atctagttta ttgaatgtga ttgcgggtcg     180
   actgccactt gatagtggga ccgtgacgat cggtgagacc gtcaagatgg cctattatac     240
   tcaacagacc gaaccgattc cgggcgacaa gcgcatcatt aattacttgc aagatgtcgg     300
   tgagacggtc ttgaataagc agggcgagca tgtttcagtg actgagttac tagaagagtt     360
   tctgttccca cgttcgatgc acggcacgct gattcgcaag ttatctgggg gcgaacagcg     420
   ccggctatac ttactgaagt tgttgatgca acagccgaac gtcctcttat tggacgaacc 480
   gactaatgat ttggatattg gtacgttaac ggtgttggag aactacctcg atgattttgc 540
   cgggaccgtc atcactgttt cccatgaccg ctattttttg gacaaggtcg gcacgaaact 600
   actgattttt gatggtcagg ggcatattga acgctattct ggccgtttct ccagttattt 660
   gaaggatcaa aaagacgcgg ccaagcccgc agccaaggcc actgcgacaa aaactacggc 720
   taagccggtc acggatgact cgaccgcacc attggccaag aaagccaaag tcaagctgac 780
   ttacgcggaa cagctcgaat acgacaagat cgaaggggtt atcgagcaat tggatagcca 840
   taagagtgag atcgaagcgg cgatggctgc caatgccagt gattatggca agttggctga 900
   tttgcaaaaa gaattgacta agacggaaca aacgattgat gagaagatgg atcgctggga 960
   ctacctcagc cagtatgcag aagcctgaaa ggatgatcgc 1000
<210> 4
   <211> 1000
   <212> DNA
   <213> Lactobacillus salivarius UCC118
<400> 4
   ttgatattaa tttaggacaa caaagattgg gaaagaaagt attagaatta aaagatgcaa 60
   gtttaacaat tggaaatcat aaaataattg aagatttcaa tttgctaatc caagctggag 120
   acagaatcgg tattacaggc gttaatggtg ccgggaaatc tagtttttta aatgctttat 180
   caggtgaatt gccattagat tcaggtattt tgacaatcgg tgaaactgta aagatggctt 240
   attatcgaca acagacggaa gagattcctg aagataaaag aattattagt tatttaaacg 300
   aagttggtca aaatattgta aataaagatg gtgagcgaat tagcacaact caattattgg 360
   aacaatttct atttccaaga tttatgcatg gaacattgat tcgcaaactc tctggcggtg 420
   aaaaaagacg cttgtatctt ttgaaattat taatgtctca acctaatgtt ttattattgg 480
   atgaaccaac taacgattta gatataggaa cattaacagt tttggaagat tatttagata 540
   actttaacgg aactgttatt accgtatcac atgacagata ttttcttgat aaggtagctg 600
   attcattata tatctttgaa ggaaatgcaa aaatcaagca ttatgtcgga atgtttacag 660
   attatctgaa gaatgctgag aatgaagcag ttaagactaa aaaacaagta tctgcaacaa 720
   aagtagaaaa gacagatagt gcagaagaca aagttaaaaa gaaaaccaag ttaacttacg 780
   ctgaaaagat ggaatatgaa aaattagaat cagaaatcga taagcttgaa aatgataaag 840
   caagtttaga agaagaaatg caacatgttg atggggctga ttatactaaa ttagcaagtt 900
   tacagcaaca aattgatgaa ttagatgaag atattatgga aaaagttcaa cgttgggacg 960
   aacttagtca gtatgttgat taataggaag gagaacaccg 1000
<210> 5
   <211> 1000
   <212> DNA
   <213> Streptococcus mutans UA 159
<400> 5
   aaatgacgag ccaataatta tggctctttt ttataaaaaa ccaatatatg tcttgacaca     60
   tgtaaaaaac gttgtataat gatttcaaac attaattaaa aaggtggagc aaaacggtgt    120
   ctagaaaagc aagcaatatc tgggataatt ttaaatggct gtggcaagaa ggtaaaaaat    180
   taggcttttg ggggatatta caagcgctat gggaggattt ggttaaaaat aggagcctgt    240
   cgcagtggct atacctttta gctcttagtt ttccaacact ggttttagaa tttatcggtg    300
   gaacacggca cattgctggt tttgcggcag cactgacagg aattctatgt gttatctttg    360
   ttgctgaggg gcgtatcagt aattatttca tcggttttat tcatgaaatg ctctatcttt    420
   atctcagttt tgaaaatatg tattatggtg aagttttaac aaccttattt ttcactgtca    480
   tgcaatttgt cggtgcttat tattggttga ttggacatcg tgaaggacag ggaaagaaag    540
   ttgaagtaaa ggatgttaaa tcacgaaaac tgacacccct aggctggttg aaatcacttg    600
   gaattacaat tattgtttgg ttagtttttg gctttattta tcgatcaatc ggttcgcatc    660
   gccctttttg ggatagttca acagatggta ccaattggag cggtcagttt cttcaaacag    720
   gcatgtacag tgaacaatgg ctcttttgga ttgcgaccaa tgttctcagc atttttcttt    780
   ggtggggtgc agaaccacat gttatgctca tgtatattat ttatatgatt aatagtattg    840
   ttggttgggt aaaatgggaa cgtgatctta gaataactca agaacagtta gcttaaataa    900
   ggagtttaaa tacaatagac aaaatgcatt tgagtttttt caatgcattt tttttagaaa    960
   tttgataaaa taagaaaagt atgaacaata agcgagaaaa                         1000
<210> 6
   <211> 1000
   <212> **DNA**
   <213> Lactobacillus acidophilus NCFM
<400> 6
   ggtgaagtaa tgattgaata tgtttgggca gaagataaag aaaaaaatat tggcttgaat 60
   ggacatttac catggtattt gccggctgat atgaagcatt ttaaagaagt aacaattaat 120
   catccaataa ttatgggaag aaaaacattt gaaagttttc ctaatttgtt acctaaaaga 180
   aaacatattg ttttaactca taatgaagag ctaaaaaata aatatcaaaa taatgatcaa 240
   gtgactattt tacccacagt tgaagattta cataattttg tggcagaaca tcaagatgag 300
   cggatgtgtg caattggtgg agtgtcgatt tttaacgctt taatggacca agtagaagta 360
   ttagaaaaaa cggagataga tgcgattttt gaagcagata ctaaaatgcc tgaaattgat 420
   tatagccgtt ttaatttagt agctaagaag cattatgagc cggatgagaa aaataagtat 480
   ccatatactt ttttaactta taaattaaag taaaaaaagc ccgcaatttg cgagcttttt 540
   tgaatcttag gtataatatt aataaatatt aagtttctta ggtaataata tatgaatgaa 600
   gaaaaaacaa atttaaatac gggcctgaca gctgcacaag ttaagcaaaa aattgaagct 660
   ggtgaaatta ataaggccgt tgatgatcaa tttaaaacaa ataaacaaat tattgcagaa 720
   aatttattta cttactttaa cttgattttc ttagtactat cattactttt gatttttgtt 780
   ggcgcatata aggatttaac ttttttgcca gtcattgttt taaatactgt aattggaatt 840
   gtgcaagaaa ttcgtgctaa aaaaatattg aacaagttaa acgtaatgaa tgctacagat 900
   attggggctt tgcgagatgg aaaagaagta caagtaccaa tagaagagtt agttaaaggc 960
   gatattgttt tattaaaaac aggcgatcag attccagcag 1000
<210> 7
   <211> 1000
   <212> DNA
   <213> Lactobacillus plantarum WCFS1
<400> 7
   gaggccgcaa tgattgcatt gatatgggca gaagatcaaa atggcctgat tggcaaccag      60
   ggccagcttc cctggcattt accagcagac atgcagcggt tcaaagcgct gacgaccgga     120
   caccatgtcg tgatggggcg caagactttt gcgggcttta aacgcccgct cccacggcgg     180
   accaattggg tgctctcacg tcagtctgat ttaaagttgc caccggaagt ccatcaacta     240
   gcagatgtgg cggcgatcca aacactcgcg gccgcccatc cggatgaacc gatttttgtc     300
   attggtggtg cggtggtgtt tgaagccgtg ttaccggtgg ccgattattt atatcggacg     360
   cgtatcaacg caaggtttga tggtgatact tggatgccgg ccgtggatta cacgcagtgg     420
   cagctggtga gccaacaaat tgggacggtg gatgagaaaa atcaatatcc gtacgaattt     480
   gatgatttcc gccgtcgtta attcagtggc aaccgtgaaa ctcaggcaga tttctgctat     540
   actgaaagca ttaattttat tgtaacgatc gtttaaacgt tacaagtata agaaagcgtg     600
   gcaaagtatg acgaaaatca agattgtgac cgattcttcg gcaaatttga cggatgccga     660
   ggtaaaaaaa tacgatatta ccgtgattcc gttgactgtc atgatcgatg gcaccattta     720
   cgtggaagat gaaacgatta cgcgtgaaga atttattgac aagatggcaa cggctaaatc     780
   tttaccaaag acgagccaac cggcactcgg aacgttcatt gaaacgtttg ataagttagg     840
   tgcggatggc gcgagtgtga tttgtatcaa tatgctcgaa gccatttcag gaacagtgca     900
   cacggcggaa caagcggctt cgattacgaa gaccgatgtc accgtgatcg acgctcggac     960
   gaccgaccgt gcgatggcct ttcaagtttt gactgctgct                          1000
<210> 8
   <211> 1000
   <212> DNA
   <213> Lactobacillus salivarius UCC118
<400> 8
   tctgtgaggt gtgcactatg atttcatttg tatgggcaga agatcaaaaa catcaaatag     60
   gatataaagg acatttacct tggagacttc cagccgattt ggctcatttt aaagaagtta    120
   caatgggtca tccaatggta atggggaaga aaacgtttga tagttttcct ggtttattac    180
   caagacgtca acatatagtg ttaacccatg atactaactt agaagaaaag tataaagata    240
   atccccaagt agaaattatg aattctattg atgaattaac ttcttggtta gatgaaaatc    300
   agtttcaaga agttagcgtt attggtggag cgatgttatt caacttactt cttaataaag    360
   tagataaatt atataagact gaaatactaa gtgagtttaa tggcgatacg gttatgccta    420
   ctattaatta tgatgaattt aaacttgttt caaaaaaaat cggtaaggta gatgaaaaaa    480
   acaagtatcc ttatgtattt ttggaatacg aaagaaaata atttctaata aaaataatga    540
   aagatgagta agacaatttt gtagtactca tcttttttta catttttaag gcttaaatgt    600
   tgacttcaag ttaacttgaa gttttattat agatatgtaa gttcatatag ggttgatatt    660
   aaaaactcta aatttttaag agaaggatgt gctaaatatg caaaaattat caagtttaac    720
   atcgccatta gttctagcga atggtgttga aattccagga ctaggctatg gtacttacca    780
   aactccacct gaagatacat acaaagtagt atcagaagca ttatcaatcg gttatcgcca    840
   cattgacaca gctgcattat atggcaatga aagtggtgta ggtaaggctg ttaaagatag    900
   tgggttgaag cgtgaagaag tatttattac tagtaagtta tggaatactg aacgtggata    960
   cgacaaagca attgcttcct ttaataagac tttagaaaat                         1000
<210> 9
   <211> 27
   <212> PRT
   <213> Lactococcus lactis subspecies Cremoris MG 1363
<400> 9
   Met Lys Lys Lys Ile Ile Ser Ala Ile Leu Met Ser Thr Val Ile Leu
   1               5                   10                  15
   Ser Ala Ala Ala Pro Leu Ser Gly Val Tyr Ala
               20                  25